# EUROPÄISCHE PATENTSCHRIFT

(11) **EP 2 937 617 B1**
(45) Veröffentlichungstag und Bekanntmachung des Hinweises auf die Patenterteilung: **01.03.2017**
(21) Anmeldenummer: 14001479.6
(22) Anmeldetag: 24.04.2014
(51) Int. Cl.: F16M 11/08, F16M 11/20, F16M 13/02

(54) **Drehbare Verbindung mit Drehwinkelbegrenzung**
Rotatable connection with limitation of the rotational angle
Liaison rotative doté d'une limitation d'angle de rotation

(43) Veröffentlichungstag der Anmeldung: 28.10.2015
(73) Patentinhaber: Ondal Medical Systems GmbH, 36088 Hünfeld (DE)
(72) Erfinder: Oginski, Stefan, 36041 Fulda (DE); Bauditz, Ronny, 98528 Suhl (DE); Göbel, Andreas, 36132 Eiterfeld (DE); Euler, Annika, 36088 Hünfeld (DE)
(74) Vertreter: Graf von Stosch, Andreas

(56) Entgegenhaltungen:
- EP-A1- 0 392 303
- EP-B1- 2 325 541
- DE-A1- 3 808 327
- DE-A1-102008 011 129
- FR-A- 1 341 061

## Beschreibung

Die vorliegende Erfindung betrifft eine drehbare Verbindung für eine Stativvorrichtung zur Anordnung in einem Operationssaal, die einen anpassbaren Anschlagmechanismus aufweist, der zwischen einer Spindel und einer relativ zur Spindel verdrehbar um eine Drehachse gelagerten Buchse anordenbar ist und eingerichtet ist, mindestens zwei unterschiedliche relative Drehwinkel der Spindel relativ zur Buchse oder mindestens zwei unterschiedliche Drehbereiche festzulegen, wobei der anpassbare Anschlagmechanismus aufweist: einen ersten Teil, insbesondere in Form eines Anschlagrings, welcher verdrehblockierbar an der Spindel lagerbar ist und mindestens einen Anschlag aufweist; und einen zweiten Teil, welcher verdrehsicher an der Buchse vorgesehen ist; wobei der erste Teil relativ zum zweiten Teil verdrehbar gelagert ist. Die vorliegende Erfindung betrifft insbesondere eine drehbare Verbindung mit einzelnen Merkmalen des Anspruchs 1 sowie ein Tragsystem bzw. eine Stativvorrichtung mit einzelnen Merkmalen des entsprechenden weiteren unabhängigen Anspruchs.

Stative, insbesondere Deckenstative wie z.B. Deckenversorgungseinheiten, Monitorträger, oder so genannte Federarme oder Zentralachsen, weisen meist einen oder mehrere in Bezug auf eine Vertikalposition starr angeordneten oder höhenverstellbaren Träger auf, mittels welchen eine daran befestigte medizintechnische Einrichtung bewegt und positioniert werden kann, z.B. in einem Operationssaal, insbesondere auch auf einer Intensivstation. An den Stativen sind häufig Versorgungseinheiten montiert, mit denen medizinisch-elektrische Endgeräte mit den z.B. während einer Operation benötigten Medien versorgt werden können. Die Träger definieren dabei einen Aktionsradius der medizintechnischen Einrichtung, in welchem die medizintechnische Einrichtung positioniert ist. Die Träger können meist zumindest um mindestens eine drehbare Verbindung, insbesondere ein Drehgelenk verdreht werden. Wahlweise sind die Träger auch höhenverstellbar und/oder um eine zumindest annähernd horizontal ausgerichtete Achse in der Höhe verschwenkbar angeordnet. Eine Drehbewegung einzelner Träger, sei es eine absolute Drehbewegung oder eine Drehbewegung relativ zu einem anderen Träger, soll in vielen Fällen auf einen vorgegebenen Winkel begrenzt werden. Hierdurch kann z.B. vermieden werden, dass ein Träger um mehr als 360° gegenüber einem anderen Träger verdreht wird und dadurch im Träger geführte Leitungen verdrillt, eingequetscht oder sogar abgerissen werden. Eine Drehwinkelbegrenzung kann z.B. in Form eines Anschlags bereitgestellt werden, an welchem ein Träger bei einem bestimmten Verdrehwinkel, z.B. 300°, anschlägt. Der Anschlag kann dabei z.B. fest am Träger montiert sein, insbesondere in Form eines in radialer Richtung eingebrachten Sicherungsbolzens. Der Anschlag gibt dabei einen vordefinierten Drehwinkel vor. Eine solche Drehwinkelbegrenzung kann zwar sicherstellen, dass ein maximaler Drehwinkel nicht überschritten wird, hat aber meist auch den Nachteil, dass die Bewegungsfreiheit des Stativs eingeschränkt wird, also dass sich z.B. eine Versorgungseinheit des Stativs nicht mehr in beliebigen Positionen anordnen lässt. Der Aktionsradius des Stativs wird eingeschränkt, insbesondere ohne Berücksichtigung einer bestimmten Raumsituation. Deshalb muss in jedem Einzelfall erwogen werden, durch welchen Anschlag die Drehwinkelbegrenzung definiert werden kann oder soll. Die richtige Auslegung der Drehwinkelbegrenzung, insbesondere eine adäquate Positionierung des Anschlags, kann jedoch bereits bei der Herstellung eines jeweiligen Stativs Schwierigkeiten mit sich bringen, insbesondere dann, wenn noch nicht geklärt ist, an welchem Ort das Stativ jeweils eingesetzt werden soll. Daher sind Drehwinkelbegrenzungen praktisch, mittels welchen ein Drehwinkel oder eine Drehposition nachträglich angepasst werden kann.

Eine Vorrichtung mit einem einstellbaren Drehwinkel gemäss dem Oberbegriff des Anspruchs 1 ist aus der EP 2 325 541 B1 bekannt.

In der EP 2 325 541 B1 wird ein zweiteiliger anpassbarer Anschlagmechanismus beschrieben, bei welchem ein ringförmiges Teil selektiv außen um einen Umfang eines ersten Trägers bzw. eines Gelenks des ersten Trägers herum positioniert werden kann, und das ringförmige Teil weist eine Vielzahl von stirnseitig angeordneten Ausnehmungen oder Vorsprünge auf, mittels welchen es relativ zum ersten Träger auf einfache Weise in unterschiedlichen Drehwinkelpositionen anordenbar ist. Am ringförmigen Teil ist ferner ein Anschlag angeordnet, an welchem ein zweiter Träger anschlagen kann. Mittels des ringförmigen Teils kann ein Drehwinkel der beiden Träger relativ zueinander eingestellt werden. Der Anschlagmechanismus ist dabei innerhalb von einem Bund des zweiten Trägers angeordnet. Das ringförmige Teil kann durch Eingriff eines Werkzeugs in eine an einer Außenmantelfläche des ringförmigen Teils umlaufende Nut angehoben werden, um das ringförmige Teil relativ zum ersten Träger in einer gewünschten Drehwinkelposition zu positionieren. Ferner ist ein weiteres ringförmiges Teil am ersten Träger vorgesehen, welches relativ zum ringförmigen Teil positioniert werden kann. Die beiden ringförmigen Teile sind innerhalb vom Bund angeordnet und werden vom Bund radial außen umschlossen und abgedeckt. Im Bund ist ein in radialer Richtung eingebrachter Sicherungsbolzen angeordnet, welcher in einen zwischen den beiden ringförmigen Teilen gebildeten Zwischenraum eingereift. Durch die relative Drehposition des ersten Teils relativ zum zweiten Teil wird die Ausdehnung des Zwischenraums in Umfangsrichtung definiert. Über die Ausdehnung des Zwischenraums in Umfangsrichtung kann der Winkelbereich definiert werden, in welchem die beiden Träger relativ zueinander verdreht werden können. Der Anschlagmechanismus ist dabei im Wesentlichen am ersten Träger angeordnet und wirkt über den radial eingebrachten Sicherungsbolzen mit dem zweiten Träger zusammen.

In der DE 38 08 327 A1 wird ein Stoppmechanismus beschrieben, bei welchem ein Gewindebolzen in radialer Richtung in einer Gewindebohrung verlagert werden kann, um unterschiedliche Drehwinkelpositionen einzustellen.

Es ist eine Aufgabe der vorliegenden Erfindung, eine drehbare Verbindung bereitzustellen, mittels welcher ein Drehwinkel bzw. Dreh(winkel)bereich auf einfache Weise einstellbar ist. Insbesondere besteht die Aufgabe auch darin, eine Stativvorrichtung mit einer Drehwinkelbegrenzung bereitzustellen, bei welcher einzelne Träger der Stativvorrichtung dank einer einstellbaren drehbaren Verbindung auf flexible Weise in einem Operationssaal positioniert werden können.

Diese Aufgabe wird gelöst durch eine drehbare Verbindung für eine Stativvorrichtung zur Anordnung in einem Operationssaal, umfassend einen anpassbaren Anschlagmechanismus, der zwischen einem ersten Verbindungsbauteil (insbesondere einem Verbindungsbauteil der drehbaren Verbindung) und einem relativ zum ersten Verbindungsbauteil verdrehbar um eine Drehachse gelagerten zweiten Verbindungsbauteil (insbesondere einem Verbindungsbauteil der drehbaren Verbindung) anordenbar ist und eingerichtet ist, mindestens zwei unterschiedliche relative Drehwinkel der Verbindungsbauteile relativ zueinander oder mindestens zwei unterschiedliche Drehbereiche festzulegen, wobei der anpassbare Anschlagmechanismus aufweist:
- einen ersten Teil, welcher verdrehblockierbar an dem ersten Verbindungsbauteil lagerbar ist und mindestens einen Anschlag aufweist;
- einen zweiten Teil, welcher verdrehsicher am zweiten Verbindungsbauteil vorgesehen bzw. anordenbar ist;
wobei der erste Teil relativ zum zweiten Teil verdrehbar gelagert ist;
wobei der anpassbare Anschlagmechanismus eine Anschlageinrichtung mit mindestens einem Gegenanschlag aufweist, welche axial (also in Bezug auf die axiale Richtung der Drehachse) zwischen den beiden Teilen angeordnet ist und dadurch mit den beiden Teilen zusammenwirken kann, wobei der mindestens eine Gegenanschlag mit dem mindestens einen Anschlag korrespondiert und wobei die Anschlageinrichtung eingerichtet ist, mittels des mindestens einen Gegenanschlags die unterschiedlichen relativen Drehwinkel oder Drehbereiche festzulegen. Hierdurch kann eine auf einfache Weise einstellbare drehbare Verbindung bereitgestellt werden. Das Einstellen kann z.B. durch axiales Verlagern und Verdrehen der Anschlageinrichtung erfolgen, insbesondere auf manuelle Weise. Die separate Anschlageinrichtung kann in unterschiedlichen Verdreh-Positionen zwischen den beiden Teilen angeordnet werden. Ein in radialer Richtung angeordneter Stift oder Sicherungsbolzen ist nicht erforderlich. Vielmehr können die Komponenten in axialer Richtung zueinander positioniert werden. Die Gegenanschläge können an der Anschlageinrichtung in vorgegebenen Positionen befestigt sein. Hierdurch ist es auch möglich, unterschiedliche Drehbereiche festzulegen, also z.B. einen Drehbereich von Kompass-Nord (also der geografischen Nord-Richtung) um z.B. 360° im Uhrzeigersinn und entgegen dem Uhrzeigersinn, oder einen Drehbereich ausgehend von Kompass-Ost um 360°, oder einen Drehbereich von Kompass-Nord um 400°. Dadurch kann der Aktionsradius z.B. einer Stativvorrichtung auch in Bezug auf eine Anordnung in Wandnähe oder in einer Ecke angepasst und eingestellt werden. Der (absolute/maximale) Betrag des Drehwinkels ist durch die geometrische Ausführung des Anschlagmechanismus, insbesondere der Anschlageinrichtung, bevorzugt fest vorgegeben, z.B. mit 360°, 400° oder 420°. Der Startpunkt der Drehbewegung ist einstellbar.

Durch eine Anordnung des Anschlagmechanismus sowohl am ersten Verbindungsbauteil als auch am zweiten Verbindungsbauteil kann eine Anschlageinrichtung vorgesehen werden, die auf einfache Weise zwischen den beiden Verbindungsbauteilen angeordnet und umpositioniert werden kann, insbesondere durch axiale Verlagerung des ersten Teils relativ zum zweiten Teil.

Mittels einer Anschlageinrichtung, die einen oder mehrere Gegenanschläge aufweist, kann die Anzahl der Komponenten gering gehalten werden. Bevorzugt ist der gesamte Anschlagmechanismus nur aus drei Komponenten aufgebaut, insbesondere dem ersten Teil, dem zweiten Teil und der Anschlageinrichtung.

Die als verdrehblockierbare Anordnung beschriebene Verbindung oder Lagerung des ersten Teils zum bzw. am Verbindungsbauteil kann dabei z.B. durch zwei aneinander schlagende oder in Eingriff miteinander bringbare Vorsprünge bereitgestellt werden, also eine formschlüssige Verbindung. Der erste Teil ist um die Drehachse verdrehbar angeordnet, insbesondere zusammen mit dem ersten Verbindungsbauteil. Der erste Teil kann beim Eingriff zusammen mit dem ersten Verbindungsbauteil verdreht werden, was z.B. durch einen Anschlag in Form eines Bolzens oder Sicherungsstiftes sichergestellt werden kann. Eine verdrehblockierbare Anordnung kann also eine Anordnung umfassen, bei welcher zwischen dem jeweiligen Teil und dem jeweiligen Verbindungsbauteil zwar eine relative Drehbewegung möglich ist, die jedoch durch irgendeinen Anschlag ab einem bestimmten Drehwinkel beschränkt ist. Sobald das Teil am Anschlag zur Anlage kommt, ist eine relative Drehbewegung zwischen dem Teil und dem Verbindungsbauteil dann in der entsprechenden Drehrichtung nicht mehr möglich. Mit anderen Worten: bei einer verdrehblockierten Anordnung kann der erste Teil zumindest in einer Drehrichtung nicht weiter um das erste Verbindungsbauteil gedreht werden. Mittels einer verdrehblockierbaren Anordnung kann ein Drehwinkelbereich, insbesondere mit einem Drehwinkel größer 360°, eingestellt werden. Eine verdrehblockierbare Anordnung kann dabei auch eine verdrehsichere Anordnung umfassen, also z.B. eine Nut-Feder-Verbindung.

Die als verdrehsichere Anordnung beschriebene Verbindung oder Lagerung des zweiten Teils zum bzw. am Verbindungsbauteil kann dabei z.B. durch eine Nut-Feder-Verbindung bereitgestellt werden, also eine Verbindung, die nur eine einzige Relativposition der beiden Bauteile zueinander definiert. Eine verdrehsichere Anordnung, Verbindung oder Lagerung kann dabei auch eine Anordnung umfassen, bei welcher der zweite Teil (einstückig) als ein integraler Teil des Verbindungsbauteils ausgebildet ist. Insbesondere kann der zweite Teil in ein als Buchse ausgebildetes zweites Verbindungsbauteil integriert sein.

Bevorzugt ist der erste Teil zwar verdrehblockierbar am ersten Verbindungsbauteil gelagert, dies jedoch bevorzugt nur in Hinblick auf eine Drehbewegung. Mit anderen Worten: die verdrehblockierbare Anordnung bringt nicht notwendigerweise eine vordefinierte Axialposition mit sich. Vielmehr ist der erste Teil in axialer Richtung bevorzugt am zweiten Verbindungsbauteil gelagert, insbesondere mittels der Anschlageinrichtung und/oder dem zweiten Teil. Dabei kann das erste Verbindungsbauteil in axialer Richtung bevorzugt am zweiten Verbindungsbauteil axial positioniert werden, oder umgekehrt, z.B. mittels eines Wellensicherungsrings.

Als drehbare Verbindung ist dabei bevorzugt eine Anordnung zu verstehen, mittels welcher eine Verdrehung zweier Bauteile zueinander um einen vorgebbaren Winkel sichergestellt werden kann. Die drehbare Verbindung ist z.B. eine Verbindung zwischen einer Buchse und einer Spindel, wobei die drehbare Verbindung nicht notwendigerweise die Buchse und Spindel umfasst, sondern z.B. nur die daran vorgesehenen Lager oder Lagerflächen dafür. Die drehbare Verbindung weist bevorzugt mindestens ein Drehgelenk auf oder bildet einen Teil des Drehgelenks. Als Drehgelenk ist dabei bevorzugt ein Gelenk zu verstehen, welches zumindest eine Drehung um eine oder mehrere Drehachsen ermöglicht, wobei auch ein translatorischer Freiheitsgrad verwirklicht sein kann. Das Drehgelenk ist bevorzugt an der Schnittstelle zwischen zwei einzelnen Trägern angeordnet, kann jedoch auch einen einzelnen Träger in mehrere Abschnitte untergliedern. Das Drehgelenk kann z.B. an der Schnittstelle zwischen einer Spindel und einer Buchse vorgesehen sein.

Als Stativvorrichtung ist dabei bevorzugt eine Vorrichtung zum Halten, ortsfesten Anordnen und/oder Verlagern mindestens einer medizintechnische Einrichtung zu verstehen, die an einer Wand (in einem Wandlager) oder einer Decke oder auch am Boden eines Operationssaals oder irgendeines anderen Raumes für medizinische Zwecke fest montiert werden kann, also z.B. ein Deckenstativ. Die Stativvorrichtung ist dann nicht vollkommen frei im Operationssaal verlagerbar, sondern kann nur in einem bestimmten Aktionsradius verlagert werden, insbesondere relativ zu einem an einer Decke oder Wand des Operationssaals angeordneten Befestigungspunkt bzw. Montagepunkt. Die Stativvorrichtung kann als an einer Decke montierte Deckenversorgungseinheit ausgebildet sein und eine oder mehrere Versorgungskonsolen aufweisen, welche an einem oder zwei Tragarmen gelagert und positionierbar ist. Die Stativvorrichtung kann auch als Monitorträger ausgebildet sein. Die Stativvorrichtung kann auch als so genannter, insbesondere an einer Wand montierter Federarm ausgebildet sein und z.B. eine Leuchte aufweisen. Die Stativvorrichtung kann auch als so genannte, insbesondere an einer Decke montierte Zentralachse ausgebildet sein und eine Mehrzahl von Tragsystemen mit jeweils mindestens einem Träger aufweisen, an welchem z.B. ein Monitor oder eine Leuchte gelagert ist. Die Stativvorrichtung muss jedoch nicht notwendigerweise fest an einer Wand montiert sein, sondern kann auch auf einem fahrbaren Unterbau montiert sein. Der fahrbare Unterbau kann z.B. mittels Bremsen ortsfest im Raum positioniert werden. Auch in diesem Fall ist ein anpassbarer Anschlagmechanismus zweckdienlich.

Als anpassbarer Anschlagmechanismus ist dabei bevorzugt jede Einrichtung zu verstehen, welche einen Drehwinkel und/oder Drehbereich eines Trägers begrenzen kann, insbesondere relativ zu einem weiteren Träger oder relativ zu einer fest im Raum positionierten (fiktiven) Drehachse, z.B. eine durch einen fest angeordneten Befestigungspunkt an einer Wand eines Raumes verlaufende Drehachse. Bevorzugt weist der anpassbare Anschlagmechanismus zumindest auch eine formschlüssig ausgebildete Verbindung auf oder ist für Formschluss ausgebildet. Der anpassbare Anschlagmechanismus kann zusätzlich auch kraftschlüssig wirken.

Als Drehbereich ist dabei bevorzugt ein Winkelbereich zu verstehen, in welchem ein Träger relativ zu einem weiteren Träger oder zu einer Wand verdreht werden kann. Der Winkelbereich kann z.B. 330° oder auch mehr als 360° betragen. Der Winkelbereich kann konstant groß sein, aber z.B. in Bezug auf unterschiedliche Umfangspositionen festgelegt werden, also z.B. von 0° bis 300° in Bezug auf eine Nord-Richtung, oder von 30° bis 330° in Bezug auf die Nord-Richtung. Der Drehbereich kann durch die unterschiedlichen Drehwinkelpositionen definiert werden.

Als erster Teil ist dabei bevorzugt ein Teil zu verstehen, welcher irgendwie verdrehblockierbar an die Drehbewegung des ersten Verbindungsbauteils (z.B. einer Spindel) gekoppelt ist und bevorzugt formschlüssig mit dem ersten Verbindungsbauteil zusammenwirkt. Der erste Teil ist dabei bevorzugt in axialer Richtung relativ zum ersten Verbindungsbauteil verlagerbar. In Umfangsrichtung ist eine relative Verlagerung zueinander blockiert oder ab einem gewissen Drehwinkel blockierbar. Der erste Teil kann z.B. ringförmig ausgebildet sein und dann als Anschlagring bezeichnet werden, welcher mindestens einen Anschlag definiert. Als Anschlag ist dabei bevorzugt irgendein insbesondere auch in axialer Richtung hervorstehender Vorsprung oder Absatz zu verstehen.

Als zweiter Teil ist dabei bevorzugt ein Teil zu verstehen, welcher verdrehsicher an die Drehbewegung des zweiten Verbindungsbauteils (z.B. einer Buchse) gekoppelt ist und z.B. formschlüssig mit dem zweiten Verbindungsbauteil zusammenwirkt, insbesondere drehsynchron. Mit anderen Worten ist der zweite Teil derart am zweiten Verbindungsbauteil vorgesehen, dass der zweite Teil und das zweite Verbindungsbauteil jedenfalls dieselbe Drehbewegung ausführen. Die Position des zweiten Teils relativ zum zweiten Verbindungsbauteil ist dann vordefiniert und auch nicht veränderbar. Der zweite Teil kann durch das zweite Verbindungsbauteil gebildet sein, z.B. angegossen sein. Bevorzugt ist der zweite Teil ortsfest am zweiten Verbindungsbauteil vorgesehen, also auch axialfest, d.h. in axialer Richtung relativ zum zweiten Verbindungsbauteil nicht verlagerbar. Der zweite Teil ist bevorzugt nur mit dem zweiten Verbindungsbauteil verbunden oder dadurch gebildet und ist vom ersten Verbindungsbauteil entkoppelt und wirkt nur mittels der Anschlageinrichtung und des ersten Teils indirekt mit dem ersten Verbindungsbauteil zusammen. Der zweite Teil kann z.B. ringförmig ausgebildet sein und kann mindestens eine formschlüssige Kontur in Form einer Verzahnung aufweisen, z.B. eine Sägezahnkontur, insbesondere an der Schnittstelle zur Anschlageinrichtung. Der zweite Teil kann dann als Zahnkranz bezeichnet werden. Bevorzugt sind am zweiten Teil keine einen Drehbereich definierenden Anschläge bzw. Gegenanschläge angeordnet. Derartige Anschläge sind nicht erforderlich, insbesondere weil eine relative Drehbewegung zwischen dem zweiten Teil und der Anschlageinrichtung nicht stattfinden muss bzw. soll. Bevorzugt ist der zweite Teil eingerichtet, die Anschlageinrichtung drehfest in einer einstellbaren Drehposition am zweiten Verbindungsbauteil zu lagern, so dass ein Anschlag des ersten Teils an der Anschlageinrichtung anschlagen kann, um eine entsprechend hervorgerufene Reaktionskraft von der Anschlageinrichtung auf den zweiten Teil zu übertragen. Mit anderen Worten: der erste Teil ist bevorzugt nur mittelbar mit dem zweiten Teil in Verbindung, insbesondere über die Anschlageinrichtung.

Als Anschlageinrichtung ist dabei bevorzugt ein Teil zu verstehen, welcher dazu eingerichtet ist, einen Gegenanschlag in einer relativ zu einem der Verbindungsbauteile, insbesondere relativ zum zweiten Verbindungsbauteil, ortsfesten Position bereitzustellen, wobei eine in Umfangsrichtung auf die Anschlageinrichtung ausgeübt (Verdreh-)Kraft, also ein Drehmoment, über den Gegenanschlag zwischen den Verbindungsbauteilen übertragen werden kann. Die Anschlageinrichtung ist bevorzugt eingerichtet, ein direktes Zusammenwirken zwischen dem ersten und zweiten Teil zu verhindern. Die Anschlageinrichtung ist bevorzugt zwischen dem ersten und zweiten Teil zwischengelagert und eingerichtet, ein Drehmoment zwischen dem ersten Teil und dem zweiten Teil zu übertragen. Bevorzugt erstreckt sich die Anschlageinrichtung zumindest abschnittsweise um die Drehachse herum, wobei die Anschlageinrichtung bevorzugt ringförmig ausgebildet ist und umlaufend um die Drehachse vorgesehen ist. Die Anschlageinrichtung kann dann z.B. als Stellring beschrieben werden. Als Gegenanschlag ist dabei bevorzugt irgendein Vorsprung, Absatz oder eine hervorstehende Nase zu verstehen.

Als eine Anordnung "axial zwischen" dem ersten und zweiten Teil ist dabei bevorzugt eine Anordnung zu verstehen, bei welcher der erste und zweite Teil nicht direkt miteinander gekuppelt sind, sondern nur indirekt mittels der Anschlageinrichtung. Eine Anordnung "axial zwischen" bedeutet bevorzugt, dass der erste Teil in axialer Richtung nicht in den zweiten Teil eingreifen muss, sondern dass ein Eingriff oder Zusammenwirken des ersten Teils mit dem zweiten Teil (allein) mittels der Anschlageinrichtung sichergestellt werden kann.

Als eine Drehwinkelposition ist dabei bevorzugt eine relative Verdrehposition eines Trägers gegenüber einem anderen angrenzenden Träger oder gegenüber einer fest im Raum in einer definierten Richtung ausgerichteten Achse zu verstehen. Die Drehwinkelposition kann auch in Bezug auf einen absoluten (Horizontal-)Winkel beschrieben werden, z.B. um eine (fiktive) vertikal ausgerichtete Drehachse.

Bevorzugt ist die Anschlageinrichtung eingerichtet, eine in Umfangsrichtung wirkende Drehkraft, die auf den Anschlag bzw. den Gegenanschlag ausgeübt wird, zwischen dem ersten und zweiten Teil zu übertragen, also vom ersten Teil auf den zweiten Teil und/oder vom zweiten Teil auf den ersten Teil. Mit anderen Worten: die Anschlageinrichtung ist eingerichtet, die beiden Teile aneinander zu kuppeln, insbesondere auch um einen bestimmten Drehwinkelbereich der Teile relativ zueinander zu definieren.

Gemäß einem Ausführungsbeispiel ist die Anschlageinrichtung verdrehsicher zu einem der beiden Teile, insbesondere zum zweiten Teil, an einem der beiden Teile in mindestens zwei unterschiedlichen Drehwinkelpositionen positionierbar. Hierdurch kann ein Startpunkt bzw. Ausgangspunkt eines bestimmten Drehwinkelbereichs eingestellt werden, insbesondere bei einem Drehwinkel größer als 360°. Bevorzugt ist der Gegenanschlag ortsfest an der Anschlageinrichtung positioniert. Der Gegenanschlag kann integral an der Anschlageinrichtung vorgesehen sein, d.h., die Anschlageinrichtung bildet mit dem Gegenanschlag ein einstückiges Teil. Wahlweise kann der Gegenanschlag oder mindestens ein Gegenanschlag von einer Vielzahl von Gegenanschlägen auch an der Anschlageinrichtung befestigt sein, z.B. mittels einer Verschraubung in radialer oder axialer Richtung. Dies erleichtert z.B. das Einstellen eines bestimmten Drehwinkels.

Bevorzugt ist der erste Teil in axialer Richtung entlang der Drehachse verlagerbar angeordnet. Hierdurch kann die Anschlageinrichtung zusammen mit dem ersten Teil auf einfache Weise in axialer Richtung verschoben werden, um den Drehbereich bzw. Drehwinkel einzustellen. Es ist nicht erforderlich, irgendeinen in radialer Richtung eingreifenden Bolzen oder einen den Bolzen aufnehmenden Bund zu entfernen, um die beiden Teile relativ zueinander in axialer Richtung zu verlagern. Hierbei kann der erste Teil über eine Zentrierung an einer Innenmantelfläche am zweiten Verbindungsbauteil geführt werden.

Bevorzugt ist die Anschlageinrichtung in axialer Richtung entlang der Drehachse verlagerbar angeordnet, insbesondere zusammen mit dem ersten Teil. Hierdurch kann das Einstellen der drehbaren Verbindung auf einfache Weise erfolgen. Dabei braucht z.B. nur die Anschlageinrichtung ergriffen zu werden, und der erste Teil kann zusammen mit der Anschlageinrichtung axial verschoben werden, insbesondere nach oben entgegen einer Gravitationskraft.

Bevorzugt sind der erste Teil und der zweite Teil und die Anschlageinrichtung in axialer Richtung in Reihe hintereinander angeordnet. Hierdurch kann die drehbare Verbindung, insbesondere der Ausgangspunkt bzw. Startpunkt des Drehwinkelbereichs, auf einfache Weise eingestellt werden, insbesondere nach einem axialen voneinander weg Schieben des ersten Teils und der Anschlageinrichtung.

Durch axiale Verlagerung der Teile bzw. der Anschlageinrichtung zueinander kann eine Einstellung des Drehbereichs auf einfache Weise vorgenommen werden. Auch kann auf einfache Weise ein Dämpfungselement zwischen den Teilen bzw. einem der Teile und der Anschlageinrichtung vorgesehen werden. Die Anordnung in Reihe hintereinander ermöglicht auch eine einfache Montage. Als eine Anordnung in Reihe hintereinander ist dabei eine Anordnung zu verstehen, bei welcher (abgesehen von einem möglicherweise dazwischen angeordneten Dämpfungselement) der erste Teil an der Anschlageinrichtung zur Anlage kommt, und bei welcher die Anschlageinrichtung am zweiten Teil zur Anlage kommt.

Bevorzugt ist die Anschlageinrichtung in axialer Richtung zwischen den beiden Teilen angeordnet und überlappt in axialer Richtung den zweiten Teil im Bereich einer formschlüssigen Kontur und überlappt in axialer Richtung den ersten Teil im Bereich des Anschlags. Indem der erste Teil und die Anschlageinrichtung in axialer Richtung überlappend angeordnet werden, kann der Anschlagmechanismus in der Form eines einfach aufgebauten Stecksystems bereitgestellt werden. Auch kann eine gute Stabilität der Anordnung sichergestellt werden, insbesondere da sich der erste Teil und die Anschlageinrichtung gegen ein Verkippen stabilisieren können, spezielle über die Innen- und/oder Außenmantelfläche der Anschlageinrichtung. Bevorzugt ist der erste Teil derart dimensioniert und geometrisch ausgebildet, dass der erste Teil, insbesondere der mindestens eine Anschlag, zumindest teilweise außen um die Anschlageinrichtung und/oder zumindest teilweise innerhalb von der Anschlageinrichtung anordenbar ist. Diese Anordnung ermöglicht eine Anordnung des mindestens einen Gegenanschlags an einer Außen- oder Innenmantelfläche der Anschlageinrichtung, wodurch eine Kraft in Umfangsrichtung weitergeleitet werden kann. Der Gegenanschlag kann besonders robust und massiv ausgeführt werden.

Bevorzugt erstreckt sich der erste Teil und/oder der zweite Teil zumindest abschnittsweise um die Drehachse herum, wobei der erste Teil und/oder der zweite Teil bevorzugt ringförmig ausgebildet ist und umlaufend um die Drehachse vorgesehen ist.

Gemäß einer Variante ist der erste Teil ringförmig ausgebildet und weist zwei oder mehr Anschläge auf, die gegenüberliegend zueinander angeordnet sind und in axialer Richtung von einer Scheibe des ersten Teils hervorstehen, insbesondere an einer Außenmantelfläche bzw. Kreisringfläche. Der erste Teil kann dabei einen rotationssymmetrisch ausgebildeten, insbesondere scheibenartigen Bereich aufweisen. Als Scheibe ist dabei bevorzugt ein weitgehend eben ausgebildetes Teil zu verstehen, welches sich im Wesentlichen in einer in radialer Richtung ausgerichteten Ebene erstreckt, mit einer deutlich geringeren Erstreckung in einer axialen Richtung orthogonal zu der Ebene. Eine Ausgestaltung als Scheibe hat den Vorteil, dass eine Gleitfläche auf einfache Weise an einer jeweiligen Stirnfläche der Scheibe bereitgestellt werden kann.

Gemäß einem Ausführungsbeispiel sind der erste Teil und die Anschlageinrichtung und wahlweise auch der zweite Teil in axialer Richtung am zweiten Verbindungsbauteil axial anordenbar bzw. axial positionierbar bzw. gelagert. Bevorzugt sind zumindest der erste Teil und die Anschlageinrichtung in axialer Richtung allein aufgrund der Gewichtskraft axial am zweiten Verbindungsbauteil positioniert. Diese freie Anordnung (ohne zusätzliche Befestigungsmittel) kann einen auf besonders einfache Weise einstellbaren bzw. anpassbaren Anschlagmechanismus bereitstellen.

Gemäß einem Ausführungsbeispiel ist die Anschlageinrichtung derart angeordnet, dass eine verdrehsichere Anordnung der Anschlageinrichtung am zweiten Teil (insbesondere ausschließlich) durch eine auf die Anschlageinrichtung wirkende Gewichtskraft bzw. Gravitationskraft sichergestellt ist. Hierbei muss zum Anpassen des Anschlagmechanismus lediglich eine Verlagerung der Anschlageinrichtung entgegen einer auf die Anschlageinrichtung wirkenden Gewichtskraft erfolgen, insbesondere zusammen mit dem ersten Teil. Es ist nicht erforderlich, irgendwelche radial eingebrachten Sicherungsstifte oder -schrauben zu entfernen. Vielmehr kann mittels eines Sicherungsrings eine axiale Sicherung des ersten Teils erfolgen.

Gemäß einem Ausführungsbeispiel weist der zweite Teil eine formschlüssige Kontur zum Festlegen der einzelnen Drehwinkelpositionen auf, insbesondere an einer nach innen weisenden Mantelfläche und/oder an einer in die axiale Richtung weisenden Stirnseite. Die Anschlageinrichtung weist eine damit korrespondierende formschlüssige Kontur auf, insbesondere an einer in axialer Richtung zum zweiten Teil oder zweiten Verbindungsbauteil hin weisenden Stirnseite. Hierdurch kann eine leicht zugängliche Steckverbindung bereitgestellt werden, mittels welcher der Anschlagmechanismus eingestellt bzw. angepasst werden kann.

Als eine formschlüssige Kontur ist dabei bevorzugt eine Verzahnung bzw. Zahnkontur oder eine Kontur mit regelmäßigen Absätzen oder Vorsprüngen zu verstehen. Dabei ist die Form eines einzelnen Zahns weitgehend beliebig. Bevorzugt weist der einzelne Zahn die Form eines Quaders bzw. im Querschnitt gesehen die Form eines Rechtecks auf. Die formschlüssige Kontur ist nicht notwendigerweise ausschließlich formschlüssig, sondern kann auch kraftschlüssig sein. Die formschlüssige Kontur ist bevorzugt nicht stoffschlüssig, um sicherzustellen, dass der mindestens eine Gegenanschlag reversibel und beliebig häufig in unterschiedlichen Drehwinkelpositionen positionierbar ist.

Bevorzugt ist die formschlüssige Kontur des zweiten Teils in einer axialen Richtung zumindest annähernd parallel zur Drehachse derart zugänglich, dass die Anschlageinrichtung mit der korrespondierenden formschlüssigen Kontur in der axialen Richtung auf den zweiten Teil aufsteckbar ist. Dies erleichtert sowohl die Montage als auch das Einstellen.

Bevorzugt sind die formschlüssige Kontur des zweiten Teils und die formschlüssige Kontur der Anschlageinrichtung jeweils als Zahnkranz ausgebildet, wobei Zähne des Zahnkranzes bevorzugt in einer axialen Richtung zumindest annähernd parallel zur Drehachse hervorstehen. Als Zahnkranz ist dabei bevorzugt eine in Bezug auf die Drehachse rotationssymmetrisch ausgebildete Kontur mit einer Vielzahl einzelner Zähne zu verstehen, bei welcher die Zähne in einheitlichem Abstand zueinander angeordnet sind. Die Ausgestaltung als Zahnkranz liefert z.B. den Vorteil kleiner Verstellschritte, denn umso mehr Zähne vorgesehen sind, desto feiner kann der Startpunkt bzw. Ausgangspunkt des Drehwinkelbereichs definiert werden, z.B. in 10°-Schritten.

Gemäß einem Ausführungsbeispiel bilden der erste Teil und die Anschlageinrichtung zusammen ein Lager, insbesondere ein Gleitlager. Dabei liegt der erste Teil auf der Anschlageinrichtung auf. Hierdurch kann der erste Teil reibungsarm relativ zur Anschlageinrichtung bzw. zum zweiten Teil verlagert werden, auch wenn an der Kontaktfläche zwischen dem erste Teil und der Anschlageinrichtung eine Normalkraft auf die Kontaktfläche wirkt. Zwar muss die Normalkraft nicht groß sein, denn sie kann z.B. der Gewichtskraft des ersten Teils entsprechen. Doch durch das Lager kann eine leichtgängige drehbare Verbindung bereitgestellt werden, und das Zusammenwirken der einzelnen Komponenten der drehbaren Verbindung kann optimiert werden. Bevorzugt ist die Anschlageinrichtung so zwischen dem ersten Teil und dem zweiten Teil angeordnet, dass der erste Teil nur mit der Anschlageinrichtung in Kontakt ist, nicht aber mit dem zweiten Teil. Der zweite Teil ist ebenfalls nur mit der Anschlageinrichtung in Kontakt. Mit anderen Worten wirkt der erste Teil (bevorzugt nur) mittels der Anschlageinrichtung mit dem zweiten Teil zusammen.

Gemäß einem Ausführungsbeispiel weist der erste Teil eine an einer Stirnseite, insbesondere an einer zur Anschlageinrichtung hin weisenden Stirnseite, angeordnete Gleitfläche auf und ist eingerichtet, mit der Gleitfläche auf der Anschlageinrichtung gleitend zu drehen. Ferner kann die Anschlageinrichtung eine an einer Stirnseite, insbesondere an einer zum ersten Teil hin weisenden bzw. vom zweiten Verbindungsbauteil weg weisenden Stirnseite, angeordnete Gleitfläche aufweisen und eingerichtet sein, den ersten Teil mittels der Gleitfläche für eine gleitende Drehbewegung um die Drehachse zu lagern. Die Gleitfläche des ersten Teils und/oder die Gleitfläche der Anschlageinrichtung kann dabei bevorzugt z.B. vollständig umlaufend als Kreisringfläche ausgebildet sein, oder auch nur abschnittsweise. Hierdurch kann das Lager für den zweiten Teil auf einfache und kostengünstige Weise bereitgestellt werden. Durch das flächige Aufliegen auf der Anschlageinrichtung kann ein robuster Anschlagmechanismus bereitgestellt werden, welcher sich auf einfache Weise manuell betätigen lässt. Es müssen keine Schrauben oder sonstigen Sicherungsmittel oder Befestigungselemente gelöst werden, allenfalls ein optional vorgesehener Sicherungsring zum axialen Sichern des ersten Teils. Das Ineinandergreifen der Komponenten, also des ersten Teils, des Anschlagrings und des zweiten Teils kann allein aufgrund der Gravitationskraft sichergestellt werden. Die flächige Lagerung auf ringförmigen Stirnflächen kann dabei eine exakte Positionierung der Komponenten relativ zueinander sicherstellen und die drehbare Verbindung sehr robust und leichtlaufend ausgestalten.

Als Gleitfläche ist dabei bevorzugt eine Fläche zu verstehen, welche einen geringen Reibungskoeffizienten für Gleitreibung aufweist, sei es aufgrund einer besonders niedrigen Rauhigkeit bzw. einer besonders glatten Oberfläche, sei es aufgrund eines reibungsarmen Materials mit Schmiereigenschaften. Als Material für die Anschlageinrichtung bzw. den Stellring kann z.B. Zinkdruckguss verwendet werden, sei es mit oder ohne Beschichtung.

Bevorzugt ist die Anschlageinrichtung als ein Stellring mit einer stirnseitig in axialer Richtung in Richtung des zweiten Teils hervorstehenden formschlüssigen Kontur ausgebildet. Als Stellring ist dabei bevorzugt ein (abgesehen von irgendwelchen Anschlägen) rotationssymmetrisches Teil zu verstehen, welches in unterschiedlichen Drehwinkelpositionen positioniert werden kann, z.B. jeweils um 15° versetzt, also z.B. in 24 unterschiedlichen Drehwinkelpositionen.

Bevorzugt ist die Anschlageinrichtung ein einstückiges, in axialer Richtung auf den zweiten Teil aufsteckbares Teil, an welchem der mindestens eine Gegenanschlag bevorzugt in radialer Richtung hervorsteht, insbesondere von einer Außen- oder Innenmantelfläche der Anschlageinrichtung. Hierdurch kann der Platzbedarf in axialer Richtung (die erforderliche Bauhöhe) gering gehalten werden und eine flache Bauform realisiert werden.

Gemäß einem Ausführungsbeispiel ist der anpassbare Anschlagmechanismus eingerichtet, einen Drehbereich mit einem relativen Drehwinkel größer 360° einzustellen, insbesondere im Bereich von 360° bis 420°. Der Drehwinkel größer 360° kann insbesondere dadurch sichergestellt werden, dass der erste Teil nicht verdrehsicher bzw. verdrehfest, sondern nur verdrehblockierbar am ersten Verbindungsbauteil angeordnet ist. Bevorzugt weist der erste Teil ein Formschlusselement auf, an welchem eine Verdrehsicherung, z.B. ein Bolzen, des ersten Verbindungsbauteils zur Anlage kommen kann. Bevorzugt ist das Formschlusselement (im Gegensatz zu dem Anschlag des ersten Teils) von der Anschlageinrichtung entkoppelt, wirkt also nicht mit der der Anschlageinrichtung zusammen, zumindest nicht hinsichtlich einer gekoppelten Drehbewegung. Dabei kann mittels der Anschlageinrichtung, insbesondere durch Festlegen der Relativposition der Anschlageinrichtung relativ zum zweiten Teil, der Startpunkt bzw. Ausgangspunkt für die Drehbewegung bzw. des Drehwinkelbereichs definiert werden. Der verhältnismäßig große Drehwinkel von mehr als 360°, insbesondere bis zu 420°, liefert z.B. den Vorteil einer großen Flexibilität. Die Anschläge können positioniert werden, ohne die Bewegungsfreiheit der Stativvorrichtung nachteilig zu vermindern. Im Gegensatz dazu kann bei vorbekannten drehbaren Verbindungen meist nur ein Dreh(winkel)bereich mit einem kleineren Drehwinkel von maximal etwa 330° eingestellt werden, oder das Einstellen des Drehwinkelbereichs ist gar nicht oder nur auf sehr aufwändige Weise möglich.

Gemäß einem Ausführungsbeispiel weist der anpassbare Anschlagmechanismus ein Dämpfungselement, insbesondere aus Elastomermaterial, auf, welches mit dem zweiten Teil und/oder der Anschlageinrichtung, insbesondere einer stirnseitig in axialer Richtung hervorstehenden formschlüssigen Kontur der Anschlageinrichtung, korrespondiert. Hierdurch kann sichergestellt werden, dass beim Aufeinandertreffen der Anschläge ein Stoß abgedämpft wird, wodurch die Lebensdauer der drehbaren Verbindung erhöht werden kann und/oder die Stativvorrichtung, insbesondere eine medizintechnische Einrichtung, geschont werden kann. Das Dämpfungselement kann ein Zurückschwingen oder Zurückfedern des Trägers bei einem schlagartigen Anschlagen der Anschläge aneinander verhindern. Als Dämpfungselement ist dabei bevorzugt ein Gummielement mit einer an die jeweilige formschlüssige Kontur angepassten Geometrie zu verstehen. Das Dämpfungselement kann z.B. die Form eines Mäanders aufweisen. Das Dämpfungselement kann als Einlage oder Mantel am zweiten Teil oder an der Anschlageinrichtung angeordnet sein, insbesondere an einer jeweiligen formschlüssigen Kontur.

Gemäß einem Ausführungsbeispiel weist die drehbare Verbindung ein Zwischenelement auf, welches in axialer Richtung gesehen zwischen dem ersten Teil, insbesondere zwischen der Anschlageinrichtung, und dem zweiten Verbindungsbauteil angeordnet ist und mindestens eine formschlüssige Kontur zur drehfesten Verbindung mit der Anschlageinrichtung oder dem zweiten Verbindungsbauteil aufweist, wobei bevorzugt an zwei gegenüberliegenden Stirnflächen des Zwischenelements jeweils eine formschlüssige Kontur angeordnet ist. Hierdurch kann der an den zwei Verbindungsbauteilen angeordnete Anschlagmechanismus noch flexibler ausgelegt werden, und kann insbesondere auch bei gegossenen Buchsen auf einfache Weise vorgesehen werden. Die formschlüssige Kontur kann eine Verdrehsicherung weiterer Elemente, z.B. eines Schleifringinnenteils, sicherstellen. Das Zwischenelement kann auch in Hinblick auf fertigungstechnische Besonderheiten Vorteile liefern. Insbesondere kann die formschlüssige Kontur am zweiten Teil auf einfachere Weise eingebracht werden, speziell an einem Ringabschnitt einer gabelförmigen Buchse. Es kann z.B. auf ein kostenaufwändiges Druckguss-Werkzeug verzichtet werden.

Als Zwischenelement ist dabei bevorzugt ein Element zu verstehen, welches sich formschlüssig sowohl mit der Anschlageinrichtung als auch mit dem zweiten Teil verdrehsicher kuppeln lässt. Das Zwischenelement ist ein optional vorgesehenes, zusätzliches Teil, an welchem eine formschlüssige Kontur auf besonders einfache Weise vorgesehen werden kann, bevorzugt an einer Stirnseite. Das Zwischenelement kann z.B. auch aus fertigungstechnischen Gründen vorgesehen werden. Eine mechanische Bearbeitung des Zwischenelements, insbesondere das Einbringen der formschlüssigen Kontur(en), kann auf einfache Weise erfolgen. Das Zwischenelement lässt sich auf einfache Weise handhaben und weist gut zugängliche Oberflächen auf. Bevorzugt ist die (jeweilige) formschlüssige Kontur durch Nuten gebildet, welche sich in radialer Richtung erstrecken. Die Nuten können sich entlang des gesamten Zwischenelements erstrecken. Wahlweise können die Nutzen abschnittsweise (als kurze Nuten) in Kombination mit Federn vorgesehen sein.

Gemäß einem Ausführungsbeispiel ist das Zwischenelement als Scheibe, insbesondere ringförmige Scheibe ausgebildet. Hierdurch kann das Zwischenelement in Reihe mit den anderen Komponenten um das erste Verbindungsbauteil herum angeordnet werden. Der flache Aufbau als Scheibe kann dabei auch einen geringen Platzbedarf in axialer Richtung sicherstellen.

Gemäß einem Ausführungsbeispiel ist das Zwischenelement keilförmig mit einer in Bezug auf die axiale Richtung uneinheitlichen axialen Abmessung bzw. Dicke ausgebildet. Hierdurch kann die drehbare Verbindung auf einfache Weise in Verbindung mit einer gegossenen Buchse verwendet werden, an welcher eine Entformungsschräge vorgesehen ist. Mittels der Keilform kann die Entformungsschräge ausgeglichen werden, so dass die beiden Teile bzw. die Anschlageinrichtung axial ausgerichtet aneinander angeordnet werden können. Mit anderen Worten: die keilförmige Geometrie ist eingerichtet, eine Entformungsschräge der Buchse auszugleichen.

Die zuvor genannte Aufgabe wird auch durch ein Tragsystem für eine Stativvorrichtung zur Anordnung in einem Operationssaal und zum Positionieren einer medizintechnischen Einrichtung im Operationssaal gelöst, welches eine erfindungsgemäße drehbare Verbindung sowie das erste Verbindungsbauteil, insbesondere in Form einer Spindel, und das zweite Verbindungsbauteil, insbesondere in Form einer Buchse, aufweist.

Als Tragsystem sind dabei bevorzugt diejenigen Komponenten der Stativvorrichtung zu verstehen, welche zumindest teilweise auch eine Funktion zum Halten und Positionieren der medizintechnischen Einrichtung übernehmen. Das Tragsystem kann eine Vielzahl von jeweils relativ zueinander verlagerbaren bevorzugt starren Armen bzw. Trägern sowie eine Vielzahl von Hebeln, Gelenken oder Lagern umfassen.

Als medizintechnische Einrichtung ist dabei bevorzugt eine Leuchte, ein Monitor und/oder eine Versorgungskonsole zu verstehen, mittels welcher Mittel für eine Versorgung eines Patienten und/oder Instrumente für einen Operateur und/oder Licht, Reinluft oder andere im Operationssaal benötigte Medien bereitgestellt werden können. Die medizintechnische Einrichtung weist bevorzugt irgendein Bedienpanel und/oder irgendeine Anzeigevorrichtung zum grafischen Darstellen von z.B. Patientendaten auf.

Gemäß einem Ausführungsbeispiel ist das zweite Verbindungsbauteil als Buchse, insbesondere gabelförmige Buchse, ausgebildet, wobei zumindest die Anschlageinrichtung und der zweite Teil und bevorzugt auch der erste Teil in der Buchse angeordnet sind, insbesondere zwischen zwei Ringabschnitten der Buchse, bevorzugt in einem der beiden Ringabschnitte, wobei die drehbare Verbindung bevorzugt ein Zwischenelement aufweist, welches in die Buchse, insbesondere in einen der beiden Ringabschnitte, eingelegt ist.

Hierdurch kann eine drehbare Verbindung bereitgestellt werden, deren Anschlageinrichtung gut zugänglich ist, was das Einstellen des Drehwinkels bzw. Drehwinkelbereichs erleichtert. Die einzelnen Komponenten können auf einfache Weise in die Buchse eingelegt werden, insbesondere von der Seite in radialer Richtung. Dabei kann auch ein zusätzliches Zwischenelement in die Buchse, insbesondere in einen der beiden Ringabschnitte, eingelegt werden, insbesondere um eine Entformungsschräge auszugleichen und/oder eine einfache oder kostengünstige Herstellung der formschlüssigen Konturen zu ermöglichen. Die einzelnen Komponenten können auch auf einfache Weise in axialer Richtung relativ zueinander verlagert werden, um den Drehwinkel bzw. Drehbereich einzustellen.

Durch das Zwischenelement kann auch eine in axialer Richtung besonders flache Bauform der drehbaren Verbindung sichergestellt werden, was z.B. bei Zentralachsen vorteilhaft ist, welche in axialer Richtung meist bereits eine beträchtliche Ausdehnung aufweisen.

Die zuvor genannte Aufgabe wird auch durch eine Stativvorrichtung zur Anordnung in einem Operationssaal und zum Positionieren einer medizintechnischen Einrichtung im Operationssaal gelöst, welche eine erfindungsgemäße drehbare Verbindung oder das zuvor beschriebene Tragsystem mit der erfindungsgemäßen drehbaren Verbindung umfasst.

In einer speziellen Ausgestaltungsform umfasst die Stativvorrichtung zur Anordnung in einem Operationssaal und zum Positionieren einer medizintechnischen Einrichtung im Operationssaal ein Tragsystem mit wenigstens einem Träger, insbesondere Tragarm, mit einer Buchse, die (relativ zu einem ortsfest angeordneten Teil der Stativvorrichtung oder zu einem weiteren Träger der Stativvorrichtung) verdrehbar um eine Drehachse an einer Spindel an einer drehbaren Verbindung, insbesondere einer erfindungsgemäßen drehbaren Verbindung, gelagert ist, wobei die drehbare Verbindung einen anpassbaren Anschlagmechanismus aufweist, der zwischen der Spindel und der relativ zur Spindel verdrehbar um die Drehachse gelagerten Buchse angeordnet ist und eingerichtet ist, mindestens zwei unterschiedliche relative Drehwinkel der Buchse relativ zur Spindel oder mindestens zwei unterschiedliche Drehbereiche festzulegen, wobei der anpassbare Anschlagmechanismus aufweist:
- einen ersten Teil, insbesondere einen Anschlagring, welcher verdrehblockierbar an der Spindel gelagert ist und mindestens einen Anschlag aufweist;
- einen zweiten Teil, welcher verdrehsicher mit der Buchse verbunden ist;
wobei der erste Teil relativ zum zweiten Teil verdrehbar gelagert ist;
wobei der anpassbare Anschlagmechanismus einen in Richtung der Drehachse axial verlagerbar angeordneten Stellring mit mindestens einem Gegenanschlag aufweist, welcher in axialer Richtung gesehen zwischen den beiden Teilen angeordnet ist (und mit den beiden Teilen zusammenwirkt), wobei der mindestens eine Gegenanschlag mit dem mindestens einen Anschlag korrespondiert und wobei der Stellring eingerichtet ist, mittels des mindestens einen Gegenanschlags die unterschiedlichen relativen Drehwinkel oder Drehbereiche festzulegen, und wobei der erste Teil den Stellring in axialer Richtung überlappt und radial innen und außen vom Stellring angeordnet ist und/oder wobei in axialer Richtung gesehen zwischen dem Stellring und dem zweiten Teil ein Zwischenelement verdrehsicher zum Stellring und zum zweiten Teil angeordnet ist.

Hierdurch kann die Stativvorrichtung, insbesondere einzelne Träger relativ zueinander, auf flexible Weise positioniert werden. Der Gegenanschlag kann im zweiten Teil versetzt werden, um eine geeignete Drehwinkelposition festzulegen, insbesondere in Bezug auf eine spezifische Anordnung der Stativvorrichtung relativ zu weiteren Komponenten im Operationssaal.

Als Träger ist dabei bevorzugt ein Ausleger oder Tragarm zu verstehen, welcher sich in einer bestimmten Richtung erstreckt und den gewünschten Aktionsradius für die unterschiedlichen Soll-Positionen der medizintechnischen Einrichtung sicherstellen kann, insbesondere durch eine Drehbewegung um eine drehbare Verbindung. Der Träger kann wahlweise auch in der Höhe verschwenkt werden und/oder in der Höhe translatorisch verlagert werden. Der Träger kann auch eine teleskopische Vorrichtung mit einem (zusätzlichen) Bewegungsfreiheitsgrad in translatorischer Richtung entlang der Längsachse des Trägers sein. Der Träger kann zumindest teilweise z.B. durch ein Stranggussprofil, insbesondere ein Aluminium-Stranggussprofil gebildet sein.

Mittels der Anschlageinrichtung ist ein Drehbereich oder ein Betrag eines Drehwinkels der drehbaren Verbindung, insbesondere ein zulässiger relativer Drehwinkel der beiden Verbindungsbauteile zueinander definierbar.

Bevorzugt ist der zweite Teil an dem bzw. an einem der Träger im Bereich der drehbaren Verbindung angeordnet. An einem der Träger kann ortsfest eine Kontur oder ein Anschlag fixiert sein, womit der Träger in Bezug auf den anderen Träger oder in Bezug auf irgendeinen anderen ortsfest angeordneten Teil in den unterschiedlichen Drehwinkelpositionen positionierbar ist.

In den nachfolgenden Zeichnungsfiguren wird die Erfindung anhand von Ausführungsbeispielen näher erläutert. Es zeigen:
- Figur 1: in schematischer Darstellung in perspektivischer Ansicht eine drehbare Verbindung gemäß einem Ausführungsbeispiel der Erfindung;
- Figur 2: in einer perspektivischen Schnittansicht die drehbare Verbindung gemäß dem in der Fig. 1 gezeigten Ausführungsbeispiel;
- Figur 3A: in einer perspektivischen Seitenansicht die drehbare Verbindung gemäß dem in der Fig. 1 gezeigten Ausführungsbeispiel in einer Explosionsdarstellung;
- Figur 3B: in einer perspektivischen Seitenansicht die drehbare Verbindung gemäß dem in der Fig. 1 gezeigten Ausführungsbeispiel in einer weiteren Explosionsdarstellung;
- Figuren 4A, 4B und 4C: in einer teilweise geschnittenen Draufsicht die drehbare Verbindung gemäß dem in der Fig. 1 gezeigten Ausführungsbeispiel in unterschiedlichen Drehwinkelpositionen;
- Figur 5: in einer perspektivischen Seitenansicht ein Zwischenelement einer drehbaren Verbindung gemäß einem weiteren Ausführungsbeispiel der Erfindung;
- Figur 6: in einer Seitenansicht das in der Fig. 5 gezeigte Zwischenelement; und
- Figur 7: in schematischer Darstellung in perspektivischer Ansicht eine drehbare Verbindung gemäß einem weiteren Ausführungsbeispiel der Erfindung;
- Figur 8: in einer perspektivischen Schnittansicht die drehbare Verbindung gemäß dem in der Fig. 7 gezeigten Ausführungsbeispiel;
- Figur 9: in einer perspektivischen Seitenansicht die drehbare Verbindung gemäß dem in der Fig. 7 gezeigten Ausführungsbeispiel in einer Explosionsdarstellung;
- Figur 10: in einer perspektivischen Schnittansicht die drehbare Verbindung gemäß dem in der Fig. 7 gezeigten Ausführungsbeispiel in einer Explosionsdarstellung
- Figur 11: in einer perspektivischen Seitenansicht ein Dämpfungselement für eine drehbare Verbindung gemäß einem Ausführungsbeispiel der Erfindung; und
- Figur 12: in einer perspektivischen Seitenansicht eine drehbare Verbindung gemäß einem weiteren Ausführungsbeispiel der Erfindung in einer Explosionsdarstellung.

Im Zusammenhang mit der Beschreibung der folgenden Figuren wird bei einzelnen Bezugszeichen, falls sie nicht explizit im Zusammenhang mit einer bestimmten Figur erläutert werden, auf die weiteren Figuren verwiesen.

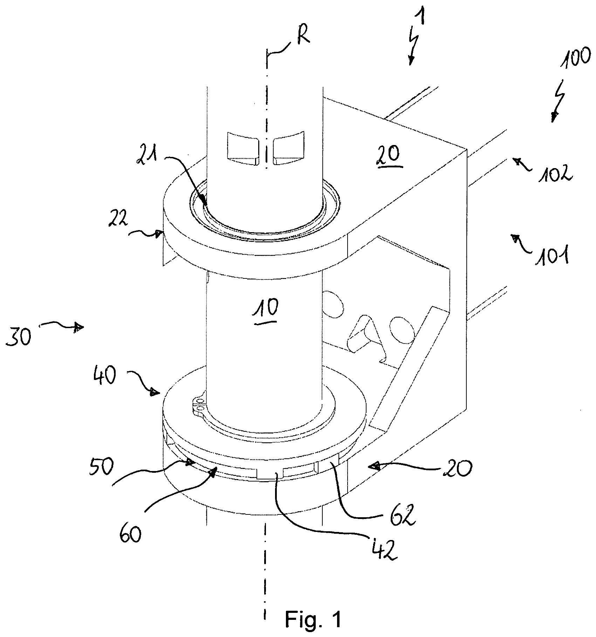

In der Figur 1 ist eine drehbare Verbindung 1 gezeigt, welche an einer Stativvorrichtung 100 um eine Drehachse R angeordnet ist. Die Stativvorrichtung 100 weist ein Tragsystem 101 mit einem ersten Träger 102 und mindestens einem weiteren Träger (nicht explizit dargestellt) auf. Die drehbare Verbindung 1 weist ein erstes Verbindungsbauteil 10, insbesondere in Form einer Spindel, und ein zweites Verbindungsbauteil 20, insbesondere in Form einer Buchse, auf. Der erste Träger 102 ist um das erste Verbindungsbauteil 10 gelagert und mit dem zweiten Verbindungsbauteil 20 verbunden. Die Buchse 20 kann dabei als gabelförmig beschrieben werden und weist zwei jeweils in einem Ringabschnitt 22 der Buchse 20 angeordnete Durchführungen 21 auf, durch welche das erste Verbindungsbauteil 10 hindurchgeführt ist. Zwischen den Ringabschnitten 22 ist eine Kavität gebildet, welche in radialer Richtung manuell zugänglich ist, insbesondere zum Anpassen eines Anschlagmechanismus 30. Die axiale Position der Buchse 20 an der Spindel 10 kann mittels eines an der Spindel befestigten Wellensicherungsrings bzw. durch eine Wellenmutter definiert werden.

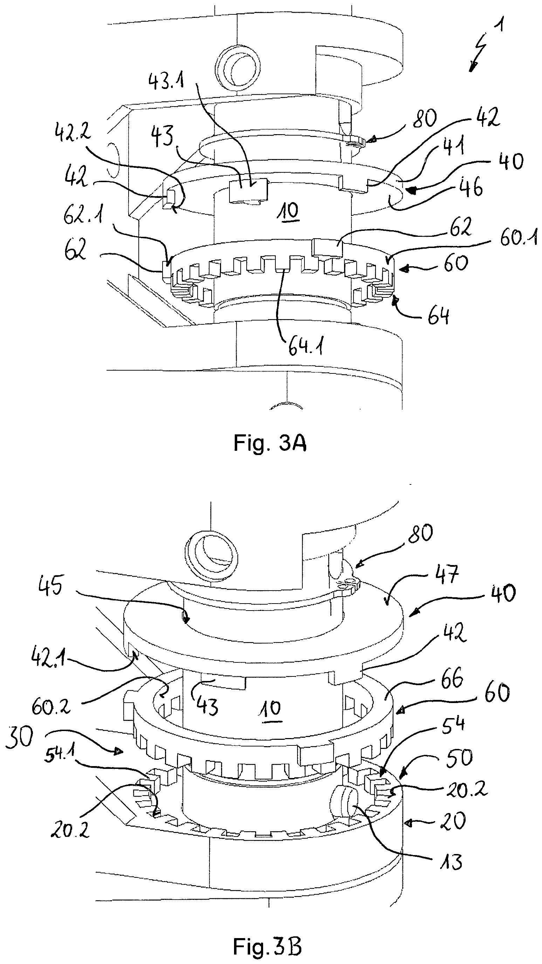

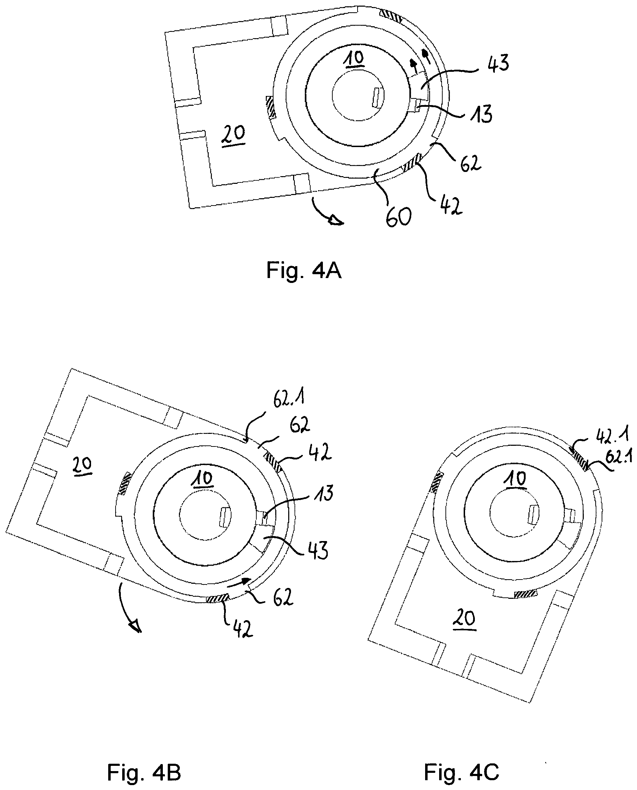

Die drehbare Verbindung 1 weist einen anpassbaren Anschlagmechanismus 30 auf, welcher zwischen der Spindel 10 und der Buchse 20 angeordnet ist. Der Anschlagmechanismus 30 weist einen ersten Teil 40 und einen zweiten Teil 50 auf. Der erste Teil 40 ist im gezeigten Ausführungsbeispiel als Anschlagring ausgebildet, und der zweite Teil 50 als Zahnkranz (vgl. Figur 3B). Der Zahnkranz 50 ist innerhalb der Buchse 20 angeordnet, insbesondere an einer Innenmantelfläche eines von zwei Ringabschnitten der gabelförmigen Buchse 20. Der Anschlagring 40 weist mehrere Anschläge 42 auf, welche an einer Stirnfläche des Anschlagrings 40 in axialer Richtung hervorstehen. Die Anschläge 42 sind ortsfest am Anschlagring 40 positioniert. Zwischen dem Anschlagring 40 und dem Zahnkranz 50 ist eine Anschlageinrichtung 60 angeordnet, welche einen Teil des anpassbaren Anschlagmechanismus 30 bildet. Die Anschlageinrichtung 60 ist im gezeigten Ausführungsbeispiel als Stellring ausgebildet Der Stellring 60 weist mehrere Gegenanschläge 62 auf, welche an einer Außenmantelfläche des Stellrings 60 angeordnet sind und in radialer Richtung hervorstehen. Die Gegenanschläge 62 sind ortsfest am Stellring 60 positioniert. Die Anschläge 42 und die Gegenanschläge 62 weisen jeweils mindestens eine ebene Seitenfläche 42.1, 62.1 (vgl. Fig. 3, 4) auf, welche sich bevorzugt zumindest annähernd in einer Ebene erstrecken, welche parallel zur Rotationsachse R verläuft. Die ebenen Seitenflächen 42.1, 62.1 bilden Anschlagsflächen, an welchen die Anschläge 42, 62 bei einer Verdrehung des Anschlagrings 40 relativ zum Stellring 60 aneinander zur Anlage kommen können. Die ebenen Seitenflächen 42.1, 62.1 korrespondieren miteinander. Der Stellring 60 kann relativ zur Buchse in unterschiedlichen Drehwinkelpositionen verdrehsicher an der Buchse positioniert werden, was im Zusammenhang mit den Figuren 3A und 3B näher beschrieben wird.

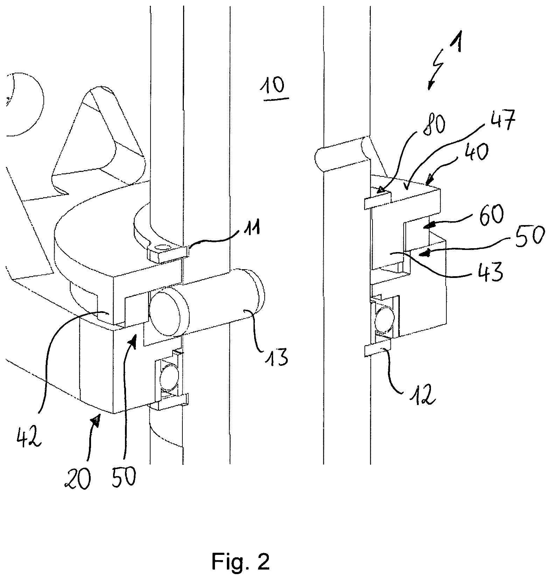

In der Figur 2 ist gezeigt, auf welche Weise der Anschlagring 40 verdrehblockierbar an der Spindel 10 gelagert sein kann. Die Spindel 10 ist zumindest abschnittsweise als Hohlwelle ausgebildet. In der Spindel 10 ist eine Verdrehsicherung 13, insbesondere ein Bolzen, angeordnet, welcher sicherstellen kann, dass sich der Anschlagring 40 nur in einem bestimmten Dreh(winkel)bereich relativ zur Spindel 10 verdrehen kann. Der Anschlagring 40 weist ein mit dem Bolzen 13 korrespondierendes Formschlusselement 43 auf, welches an einer Innenseite des Anschlagrings 40 angeordnet ist. Mittels der verdrehblockierbaren Anordnung kann ein Totwinkel überbrückt werden, welcher z.B. aufgrund der Ausdehnung des Anschlags bzw. Gegenanschlags in Umfangsrichtung entsteht. Die verdrehblockierbare Anordnung ermöglicht einen vordefinierbaren Drehbereich mit einem Drehwinkel größer 330°, oder auch größer 360°, insbesondere bis 420°. Ein verdrehblockierbarer Anschlagring 40 kann auch als ein Zwischenring bezeichnet werden, welcher einen Totwinkel überbrückt und wirkungsmäßig zwischen der Spindel 10 und den Gegenanschlägen 62 angeordnet ist.

Die Spindel 10 weist eine Nut 11 zur Aufnahme eines Sicherungsrings 80 auf. Der Sicherungsring 80 kann verhindern, dass der Anschlagring 40 in axialer Richtung nach oben verlagert wird. Der Anschlagring 40 weist eine (zweite) Stirnfläche 47 auf, welche am Sicherungsring 80 zur Anlage kommen kann. Der Sicherungsring 80 kann auf einfache Weise entfernt werden, um die drehbare Verbindung 1 zu justieren. Dazu kann der Anschlagring 40 nach oben verschoben werden, wobei daraufhin der Stellring 60 so weit nach oben verschoben wird, bis der Stellring 60 nicht mehr in die Buchse 20 bzw. den (nicht im Detail dargestellten, innen an der Buchse 20 ausgebildeten) Zahnkranz 50 eingreift, daraufhin der Stellring 60 verdreht wird, und in einer anderen Drehwinkelposition wieder nach unten verschoben wird und in Eingriff mit der Buchse 20 bzw. dem Zahnkranz 50 gebracht wird. Der Sicherungsring 80 ist jedoch nicht notwendigerweise erforderlich. Der anpassbare Anschlagmechanismus kann auch ganz ohne irgendwelche Sicherungsschrauben oder -ringe angepasst werden.

Der Stellring 60 weist einen Durchmesser auf, welcher kleiner ist als ein Teilkreis, auf welchem die Anschläge 42 angeordnet sind, und welcher größer ist als ein Teilkreis, auf welchem das Formschlusselement 43 angeordnet ist. Mit anderen Worten: der Anschlagring 40 umgreift den Stellring 60 in radialer Richtung mit dem relativ zum Stellring 60 innen liegenden Formschlusselement 43 und den relativ zum Stellring 60 außen liegenden Anschlägen 42. Der Innendurchmesser einer Innenmantelfläche des Stellrings 60 ist deutlich größer als der Außendurchmesser einer Außenmantelfläche der Spindel 10. Der Stellring 60 ist nicht an der Spindel 60 gelagert. Vielmehr kann der Stellring 60 von außen an seiner Außenmantelfläche am zweiten Teil 50 bzw. in der Buchse 20 zentriert werden. Der Anschlagring 40 kann über die Anschläge 42 über die Außenmantelfläche des Stellrings 60 zentriert werden. Eine Zentrierung an der Spindel 10 ist nicht erforderlich. Dies kann eine relative Drehbewegung bei wenig Reibung sicherstellen.

In der Figur 3A ist die drehbare Verbindung 1 in einer Anordnung gezeigt, in welcher die Drehwinkelposition eingestellt werden kann. Der Stellring 60 ist in axialer Richtung nach oben verlagert worden, nachdem der Sicherungsring 80 entfernt bzw. aus der korrespondierenden Nut gelöst wurde. Hierdurch kann eine formschlüssige Kontur 64 des Stellrings 60 aus einer korrespondierenden formschlüssigen Kontur 54 des in der Figur 3B gezeigten Zahnkranzes 50 herausgelöst bzw. in axialer Richtung herausgezogen werden. Der Stellring 60 ist in axialer Richtung entlang der Drehachse R verlagerbar angeordnet, insbesondere zusammen mit dem Anschlagring 40. Der Stellring 60 und der Anschlagring 40 sind in axialer Richtung in Reihe hintereinander angeordnet und greifen in axialer Richtung ineinander. Die formschlüssige Kontur 64 ist als Verzahnung ausgebildet, die in axialer Richtung hervorsteht. Die formschlüssige Kontur 64 weist eine Vielzahl einzelner Zähne 64.1 auf, welche in einem gleichbleibenden Abstand auf einem Teilkreis in Umfangsrichtung an einer Unterseite des Stellrings 60 angeordnet sind. Mittels der formschlüssigen Kontur 64 kann der Stellring 60 verdrehsicher zum (nicht sichtbaren) zweiten Teil bzw. Zahnkranz in mindestens zwei unterschiedlichen Drehwinkelpositionen positioniert werden.

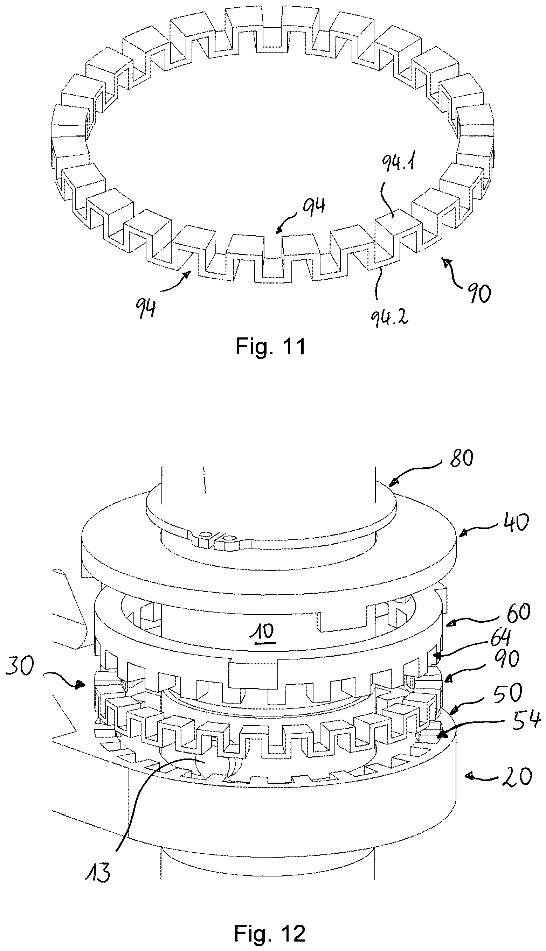

An der formschlüssigen Kontur 64 kann ein Dämpfungselement (nicht dargestellt, nur in Figur 11 und 12 gezeigt) angeordnet werden, welches zwischen der formschlüssigen Kontur 64 und der in Figur 3B gezeigten der formschlüssigen Kontur 54 des zweiten Teils 50 wirken kann. Das Dämpfungselement kann z.B. als mäanderförmiger Ring aus einem Elastomer ausgebildet sein, dessen Geometrie mit der Geometrie der Zähne 64.1 korrespondiert.

Aus der Figur 3A geht hervor, dass der Stellring 60 drei Gegenanschläge 62 aufweist (davon sind zwei sichtbar), die in einem Winkel von etwa 120° in Umfangsrichtung zueinander versetzt angeordnet sind. Ebenso sind am Anschlagring 40 drei Anschläge 42 vorgesehen, die in einem Winkel von etwa 120° in Umfangsrichtung zueinander versetzt angeordnet sind. Ferner ist das Formschlusselement 43 an einer Umfangsposition zumindest annähernd mittig zwischen zwei der drei Anschläge 42 angeordnet. Diese Anordnung der Anschläge 42, 62 und des Formschlusselements 43 relativ zueinander kann insbesondere auch eine günstige Lastverteilung sicherstellen.

In der Figur 3A ist ferner ein scheibenförmiger Abschnitt 41 des Anschlagrings 40 gezeigt. Die drei Anschläge 42 stehen in axialer Richtung vom scheibenförmigen Abschnitt 41 hervor. Die Anschläge weisen jeweils eine konkave bzw. konkav nach innen gewölbte Innenfläche 42.2 auf, mittels welcher sie an einer Außenmantelfläche 60.1 (mit zumindest annähernd demselben Krümmungsradius) des Stellrings 60 zur Anlage kommen können. Das Formschlusselement 43 weist eine konvexe bzw. konvex nach außen gewölbte Außenfläche 43.1 auf, mittels welcher das Formschlusselement 43 an einer Innenmantelfläche 60.2 (mit zumindest annähernd demselben Krümmungsradius) des Stellrings 60 zur Anlage kommen kann. Hierdurch kann eine relative Drehbewegung ohne Verkanten und durch gleitendes Aneinanderliegen und gegenseitiges Führen oder Zentrieren erfolgen.

In der Figur 3B ist gezeigt, auf welche Weise der Stellring 60 mit der Buchse 20 verkuppelt werden kann. Die formschlüssige Kontur 54 ist an einer nach innen weisenden Mantelfläche 20.2 der Buchse 20 angeordnet und steht in radialer Richtung nach innen hervor. Die formschlüssige Kontur 54 weist eine Vielzahl einzelner Zähne 54.1 auf, welche in einem gleichbleibenden Abstand auf einem Teilkreis in Umfangsrichtung an der Mantelfläche 20.2 angeordnet sind. Der Stellring 60 ist dabei in einer Kavität radial zwischen der Spindel 10 und der Buchse 20 anordenbar, und die Verdrehsicherung bzw. der Bolzen 13 ragt in eine radial zwischen der Spindel 10 und dem Stellring 60 gebildete Kavität, in welcher auch das Formschlusselement 43 verlagerbar anordenbar ist. Der Stellring 60 ist in radialer Richtung zwischen dem Formschlusselement 43 und den Anschlägen 42 anordenbar.

Aus den Figuren 3 und 4 geht hervor, dass der Anschlagring 40 mit einer (ersten) Stirnfläche 46 auf einer korrespondierenden Stirnfläche 66 des Stellrings 60 zur Anlage kommen kann und daran bei einer relativen Drehbewegung entlang gleiten kann. Die Stirnfläche 46 weist dabei einen ringförmigen Gleitflächenabschnitt (bzw. ein Lager) auf, welcher (bzw. welches) zwischen den Anschlägen 42 und dem Formschlusselement 43 angeordnet ist. Mit anderen Worten bildet der Stellring 60 ein Lager für den Anschlagring 40, insbesondere ein Gleitlager. Hierzu kann die Stirnfläche 66 z.B. auch eine Beschichtung mit einem niedrigen Reibkoeffizienten aufweisen, oder der Stellring 60 kann zumindest teilweise aus einem entsprechenden Material ausgeführt sein. Gleiches gilt für den Anschlagring 40 bzw. die Flächen 42.2 und 43.1 und 46. Die auf die Stirnfläche 66 einwirkende Kraft ist jedoch nicht groß: der Anschlagring 40 weist nur eine verhältnismäßig kleine Gewichtskraft auf. Eine Reibkraft zwischen dem Anschlagring 40 und dem Stellring 60 kann bei dieser Anordnung nahezu vernachlässigbar sein. Die Stirnflächen 46, 66 bzw. entsprechende Abschnitte können auch als Gleitflächen bzw. Gleitflächenabschnitte bezeichnet werden.

Die Funktionsweise des Anschlagmechanismus kann unter Bezugnahme auf die Figuren 4A, 4b und 4C in wenigen Worten auch wie folgt beschrieben werden: die Spindel 10 (und damit auch der in der Spindel 10 befestigte Radialbolzen 13) bildet eine feststehende Komponente, und die Buchse 20 hingegen die bewegliche Komponente. Mit einer Verlagerung eines Trägers (nicht dargestellt) wird die Buchse 20 ausgehend von der in Figur 4A gezeigten Relativposition relativ zur Spindel 10 entgegen dem Uhrzeigersinn verdreht, bis das Formschlusselement 43 des Anschlagrings (von welchem in der gezeigten Schnittansicht nur die mit Schraffur gekennzeichneten Anschläge 42 und das Formschlusselement 43 sichtbar sind) auf den Radialbolzen 13 trifft, hier entsprechend einem Verdrehwinkel von etwa 330°. Dabei ist der Stellring 60 verdrehsicher an der Buchse 20 angeordnet und dreht in demselben Maße mit wie die Buchse 20. Ab dem in der Figur 4B gezeigten Verdrehpunkt kann eine Relativbewegung zwischen dem Anschlagring und dem Stellring 60 erfolgen, insbesondere kann der Stellring 60 unter dem Anschlagring relativ zum Anschlagring gleiten und weitergedreht werden, bis der entsprechende Gegenanschlag 62mit der Seitenfläche 62.1 an der korrespondierenden Seitenfläche des Anschlag 42 des Anschlagrings anschlägt, was in diesem Ausführungsbeispiel eine relative Verdrehung von weiteren 90° ermöglicht. Die in der Figur 4C mit dem Bezugszeichen 42.1 gekennzeichnete Seitenfläche des Anschlags 42 ist dann freiliegend, und der entsprechende Gegenanschlag 62 ist mit seiner in Drehrichtung vorn liegenden Seitenfläche 62.1 an der gegenüberliegenden Seitenfläche des Anschlags 42 zur Anlage gebracht worden. In der in Figur 4C gezeigten Position wurde die Buchse 20 um mehr als eine komplette Drehung entgegen dem Uhrzeigersinn um die Spindel 10 gedreht. Diese verdrehblockierbare Kupplung zwischen Spindel 10 und Stellring 60 kann einen Drehbereich größer 360° sicherstellen, z.B. bis zu 420°. Entsprechend kann der Mechanismus in entgegen gesetzter Drehrichtung genutzt werden.

In der Figur 3B ist ferner eine Zentrierung 45 des Anschlagrings 40 angedeutet, mittels welcher der Anschlagring 40 in Bezug auf die Spindel 10 zentriert werden kann. Die Zentrierung 45 kann durch eine Innenmantelfläche oder einen Abschnitt einer Innenmantelfläche des Anschlagrings 40 gebildet sein. Dabei weist die Innenmantelfläche nicht notwendigerweise denselben Durchmesser auf wie die Außenmantelfläche der Spindel 10, sondern der Durchmesser kann auch etwas größer sein, um eine einfache relative Verschiebung bei wenig Reibung zu ermöglichen, sei es in Umfangsrichtung oder in axialer Richtung. Die Zentrierung 45 kann ferner auch durch einen nach innen weisenden Flächenabschnitt des Formschlusselements 43 gebildet sein, wodurch noch effektiver sichergestellt werden kann, dass beim axialen Verlagern des Anschlagrings 40 kein Verkanten an der Spindel 10 erfolgt. Die Zentrierung 45 muss jedoch nicht allein die Anordnung des Anschlagrings 40 sicherstellen.

Vielmehr kann sie wahlweise (zusätzlich) vorgesehen sein. Eine Zentrierung kann auch bereits allein durch die konkave Innenfläche 42.2 des Anschlagrings 40 am Stellring 60 erfolgen, wobei der Stellring 60 seinerseits am zweiten Teil zentriert sein kann. Diese Möglichkeiten der Zentrierung liefern eine einfach aufgebaute Anordnung mit exakt relativ zueinander anordenbaren Komponenten.

Bei dem gezeigten Ausführungsbeispiel kann ein bestimmter Drehbereich mit einem Drehwinkel größer 360° erzielt werden, insbesondere ein Drehwinkel im Bereich von 360° bis 420°.

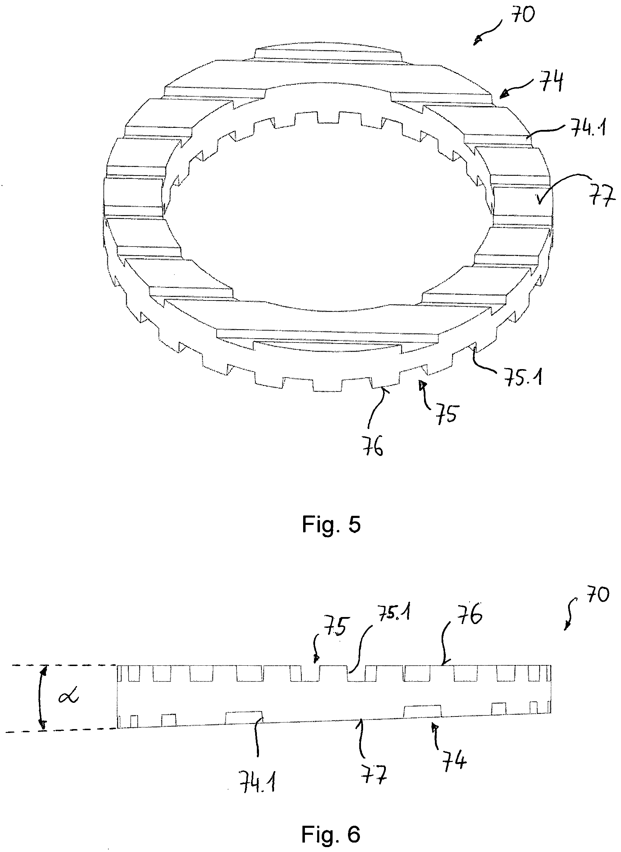

In der Figur 5 ist ein Zwischenelement 70 gezeigt, welches an einer Stirnseite (wie dargestellt an der Oberseite) eine erste formschlüssige Kontur 74 und an einer anderen Stirnseite (wie dargestellt an der Unterseite) eine zweite formschlüssige Kontur 75 aufweist. Die formschlüssigen Konturen 74, 75 weisen jeweils einzelne Nuten 74.1, 75.1 auf. Die an einer ersten Stirnfläche 76 angeordneten Nuten 75.1 erstrecken sich in radialer Richtung. Die Nuten 75.1 sind bevorzugt einen einheitlichen Winkel zueinander angeordnet, also in Umfangsrichtung gesehen in einem einheitlichen Abstand zueinander. Die an einer zweiten Stirnfläche 77 angeordneten Nuten 74.1 erstrecken sich linear geradlinig und sind bevorzugt parallel zueinander ausgerichtet. Die Nuten 74.1 weisen bevorzugt einen einheitlichen Abstand zueinander auf. Das Zwischenelement 70 ist ringförmig ausgebildet, und die Stirnflächen 76, 77 sind eben bzw. plan ausgebildet. Das Zwischenelement 70 ist als ringförmige Scheibe ausgebildet.

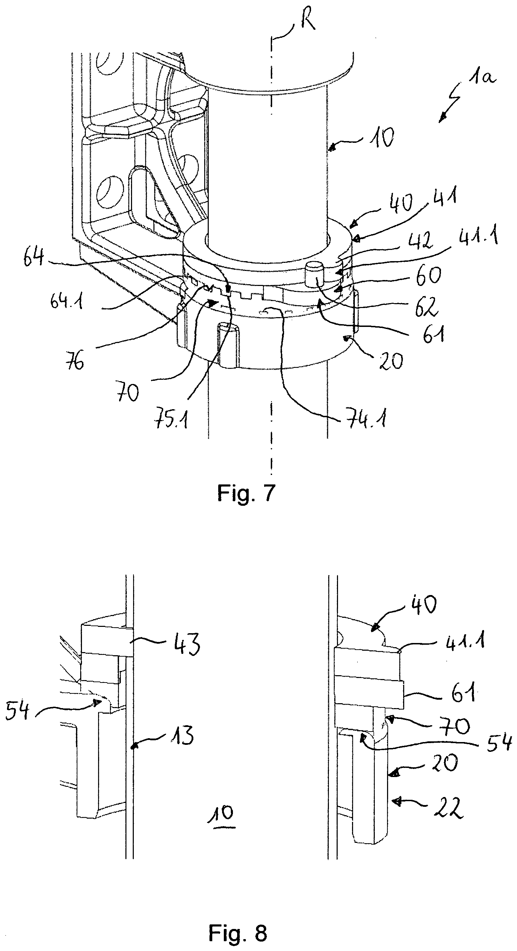

In der Figur 6 ist das Zwischenelement 70 in einer Seitenansicht gezeigt. Aus der Figur 6 geht hervor, dass die erste Stirnfläche 76 in einem Winkel α zur zweiten Stirnfläche 77 angeordnet ist, entsprechend der Neigung einer Entformungsschräge. Der Winkel α liegt bevorzugt im Bereich von 1,5°. Die Stirnflächen 76, 77 sind nicht parallel. Das Zwischenelement 70 ist keilförmig ausgebildet, insbesondere als keilförmige Ringscheibe. Hierdurch kann eine Entformungsschräge der Buchse ausgeglichen werden, was im Zusammenhang mit der Figur 7 näher beschrieben wird. Dabei entspricht die Stirnfläche 76 bevorzugt derjenigen Stirnfläche, welche mit dem Stellring 60 verkuppelt wird.

In der Figur 7 ist ein Ausführungsbeispiel gezeigt, bei welchem in Abwandlung zu dem in den Figuren 1 bis 3B gezeigten Ausführungsbeispiel das in den Figuren 5 und 6 gezeigte Zwischenelement 70 vorgesehen ist. Das Zwischenelement 70 ist in axialer Richtung gesehen zwischen der Buchse 20 und dem Stellring 60 angeordnet. Das Zwischenelement 70 ist in die Buchse 20 eingelegt. Das Zwischenelement 70 kann auf einen der Ringabschnitte 22 der Buchse 20 geschoben werden, wie aus Figur 8 ersichtlich. Hierdurch kann auch eine flache Bauform der drehbaren Verbindung 1 a realisiert werden. Dabei weist die erste Stirnfläche 76 zum Stellring 60. Die radial ausgerichteten Nuten 75.1 korrespondieren mit den einzelnen Zähnen 64.1 des Stellrings 60. Die nicht im Detail dargestellten Nuten 74.1 oder Längsrippen an der zweiten Stirnfläche korrespondieren mit einer entsprechenden formschlüssigen Kontur des zweiten Teils, welcher (nicht sichtbar) innerhalb der Buchse 20 angeordnet ist und bevorzugt durch die Buchse 20 selbst gebildet ist. Sich längs erstreckende Nuten oder Ausnehmungen können dabei auf einfache bzw. kostengünstige Weise in der Buchse 20 ausgebildet werden, insbesondere einfacher als radial ausgerichtete Nuten.

Die Buchse 20 kann z.B. als Gussteil ausgeführt sein. Dann weist die Buchse 20 bevorzugt eine Entformungsschräge auf, insbesondere auch im Bereich der formschlüssigen Kontur des zweiten Teils, welcher durch die Buchse 20 bereitgestellt werden kann. Die Entformungsschräge ist vorgesehen, um die fertig gegossene Buchse 20 aus einer Gussform entnehmen zu können. Ist die Buchse als Gussteil ausgebildet, kann auf eine Entformungsschräge nicht ohne weiteres (ohne aufwändige konstruktive Maßnahmen) verzichtet werden. Zum Ausgleich dieser Gusschräge kann das Zwischenelement 70 Stirnflächen 76, 77 aufweisen, welche in einem Winkel α entsprechend dem Winkel der Gusschräge zueinander angeordnet sind. Mit anderen Worten: das Zwischenelement 70 kann zum einen ermöglichen, dass die formschlüssige Kontur des zweiten Teils auf einfache Weise in den zweiten Teil bzw. in die Buchse eingebracht werden kann. Zum anderen kann eine streng axiale Anordnung der einzelnen Komponenten zueinander auch bei einer als Gussteil ausgebildeten Buchse sichergestellt werden. Das Zwischenelement 70 ermöglicht daher, dass die drehbare Verbindung auf einfache und kostengünstige Weise bereitgestellt und ausgelegt werden kann, selbst bei gegossenen Buchsen.

Bei dem in der Figur 7 gezeigten Ausführungsbeispiel ist am Stellring 60 an einer Außenmantelfläche des Stellrings 60 eine Lasche 61 ausgebildet, von welcher ein Gegenanschlag 62 in axialer Richtung nach oben zum Anschlagring 40 hin hervorsteht. Der Gegenanschlag 62 ist als eine Art Bolzen ausgebildet. Der Anschlagring 40 weist am scheibenförmigen Abschnitt 41 an einer Außenmantelfläche eine Lasche 41.1 auf, welche in radialer Richtung hervorsteht und einen Anschlag 42 bildet. Nichtsdestotrotz können die Komponenten 40 und 60 beim Ausführungsbeispiel der Figur 7 in Abwandlung dazu auch gemäß den in den Figuren 1 bis 3B gezeigten Varianten bereitgestellt werden. Beim Ausführungsbeispiel der Figur 7 ist der Anschlagring 40 drehfest mit der Spindel 10 verbunden. Eine relative Drehbewegung zwischen der Spindel 10 und dem Anschlagring 40 wird dadurch verhindert.

In der Figur 8 ist gezeigt, dass das Zwischenelement 70 auf einen Ringsabschnitt 22 der Buchse 20 geschoben werden kann, bevor die Buchse 20 an der Spindel 10 angeordnet wird. Dabei weist die untere (zweite) Stirnfläche 77, an welcher die parallel zueinander ausgerichteten geradlinigen Nuten 74.1 angeordnet sind, zum Ringsabschnitt 22. Am Ringabschnitt 22 können korrespondierende Nuten vorgesehen sein, welche die formschlüssige Kontur 54 bilden. Der Anschlagring 40 ist nicht nur verdrehblockierbar, sondern verdrehsicher und zudem axial verschiebbar an der Spindel 10 gelagert. Die entsprechende Verdrehsicherung 13 ist hier als eine in der Außenmantelfläche der Spindel 10 ausgebildete Nut ausgeführt, in welche das Formschlusselement 43 eingreift.

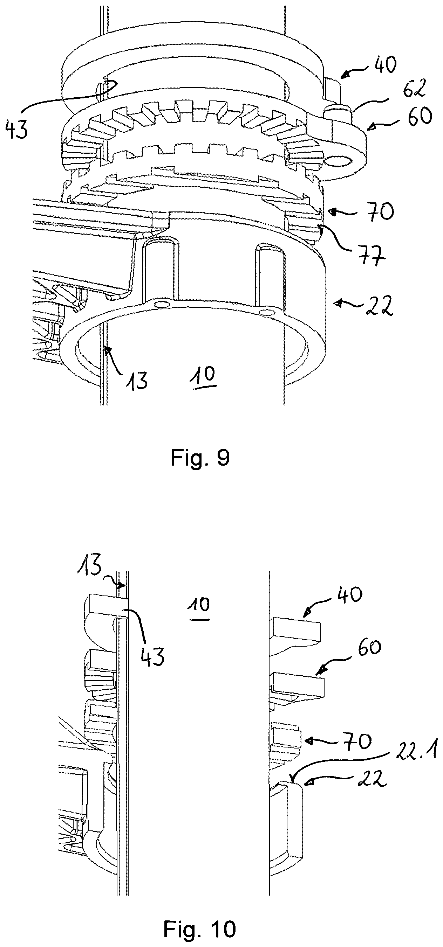

In den Figuren 9 und 10 ist eine Anordnung der Komponenten gezeigt, in welcher der Stellring 60 in einer bestimmten Drehwinkelposition relativ zum Zwischenelement 70 positioniert werden kann. Dabei ist es nicht notwendigerweise erforderlich, auch das Zwischenelement 70 axial zu verlagern. Der Ringabschnitt 22 weist eine Stirnfläche 22.1 zur Aufnahme des Zwischenelements 70 auf. Die Stirnfläche 22.1 weist in eine zwischen den beiden Ringabschnitten gebildete Kavität. An der Stirnfläche 22.1 ist die formschlüssige Kontur 54 ausgebildet. Der Ringabschnitt 22 umfasst den zweiten Teil. Der zweite Teil ist in den Ringabschnitt 22 integriert.

In der Figur 11 ist ein Dämpfungselement 90 gezeigt. Das Dämpfungselement 90 ist ein Gummielement mit einer an die jeweilige formschlüssige Kontur angepassten Geometrie. Das Dämpfungselement weist die Form eines Mäanders auf.

In der Figur 12 ist ein anpassbarer Anschlagmechanismus 30 mit einer Anordnung mit einer Spindel 10 und einer Buchse 20 vergleichbar zur in der Figur 3B gezeigten Anordnung gezeigt. Zwischen dem Stellring 60 und dem zweiten Teil 50 ist das in Fig. 11 gezeigte Dämpfungselement 90 angeordnet. Die Anordnung zwischen dem Stellring 60 und dem zweiten Teil 50 hat den Vorteil, dass keine Relativbewegung am Dämpfungselement 90 erfolgen muss. Eine formschlüssige Kontur 94 am Dämpfungselement 90 ist an beiden Stirnseiten des Dämpfungselements 90 ausgebildet. Die formschlüssige Kontur 94 weist in beiden axialen Richtungen eine Verzahnungs-Geometrie mit Zähnen 94.1 und 94.2 auf. Die formschlüssige Kontur 94 korrespondiert sowohl mit der formschlüssigen Kontur 54 des zweiten Teils 50 als auch mit der formschlüssigen Kontur 64 des Stellrings 60. Das Dämpfungselement 90 ist als Einlage bzw. als die formschlüssige Kontur 54 oder 64 umgebender Mantel am zweiten Teil 50 oder am Stellring 60 angeordnet.

### Bezugszeichenliste

- 1, 1a: drehbare Verbindung
- 10: erstes Verbindungsbauteil, insbesondere Spindel
- 11: Nut
- 13: Verdrehsicherung, insbesondere Radialbolzen oder Nut in Außenmantelfläche
- 20: zweite Verbindungsbauteil, insbesondere Buchse
- 20.2: nach innen weisende Mantelfläche der Buchse
- 21: Durchführung für erstes Verbindungsbauteil
- 22: Ringabschnitt der gabelförmigen Buchse
- 22.1: Stirnfläche
- 30: anpassbarer Anschlagmechanismus
- 40: erster Teil, insbesondere Anschlagring
- 41: scheibenförmiger Abschnitt
- 41.1: Lasche
- 42: Anschlag
- 42.1: Seitenfläche, insbesondere ebene Anschlagfläche
- 42.2: konkave bzw. konkav nach innen gewölbte Innenfläche
- 43: Formschlusselement
- 43.1: konvexe bzw. konvex nach außen gewölbte Außenfläche
- 45: Zentrierung
- 46: erste Stirnfläche, insbesondere Gleitfläche
- 47: zweite Stirnfläche
- 50: zweiter Teil, insbesondere Zahnkranz
- 54: formschlüssige Kontur
- 54.1: einzelner Zahn
- 60: Anschlageinrichtung, insbesondere Stellring
- 60.1: Außenmantelfläche
- 60.2: Innenmantelfläche
- 61: Lasche
- 62: Gegenanschlag
- 62.1: Seitenfläche, insbesondere ebene Anschlagfläche
- 64: formschlüssige Kontur
- 64.1: einzelner Zahn
- 66: Stirnfläche, insbesondere Gleitfläche
- 70: Zwischenelement
- 74: (erste) formschlüssige Kontur
- 74.1: einzelne Nut
- 75: zweite formschlüssige Kontur
- 75.1: einzelne Nut
- 76: erste Stirnfläche
- 77: zweite Stirnfläche
- 80: Sicherungsring
- 90: Dämpfungselement
- 94: formschlüssige Kontur
- 94.1: einzelner Zahn seitens des Stellrings
- 94.2: einzelner Zahn seitens des zweiten Teils
- 100: Stativvorrichtung
- 101: Tragsystem
- 102: (erster) Träger
- R: Drehachse
- α: Winkel der beiden Stirnflächen des Zwischenelementes zueinander

## Patentansprüche

1. Drehbare Verbindung (1; 1 a) für eine Stativvorrichtung (100) zur Anordnung in einem Operationssaal, umfassend einen anpassbaren Anschlagmechanismus (30), der zwischen einem ersten Verbindungsbauteil (10) und einem relativ zum ersten Verbindungsbauteil (10) verdrehbar um eine Drehachse (R) gelagerten zweiten Verbindungsbauteil (20) anordenbar ist und eingerichtet ist, mindestens zwei unterschiedliche relative Drehwinkel der Verbindungsbauteile (10, 20) relativ zueinander oder mindestens zwei unterschiedliche Drehbereiche festzulegen, wobei der anpassbare Anschlagmechanismus (30) aufweist:
- einen ersten Teil (40), welcher verdrehblockierbar am ersten Verbindungsbauteil (10) lagerbar ist und mindestens einen Anschlag (42) aufweist;
- einen zweiten Teil (50), welcher verdrehsicher am zweiten Verbindungsbauteil (20) anordenbar ist;
wobei der erste Teil (40) relativ zum zweiten Teil (50) verdrehbar gelagert ist;
**dadurch gekennzeichnet, dass** der anpassbare Anschlagmechanismus (30) eine Anschlageinrichtung (60) mit mindestens einem Gegenanschlag (62) aufweist, welche axial zwischen den beiden Teilen (40, 50) angeordnet ist, wobei der mindestens eine Gegenanschlag (62) mit dem mindestens einen Anschlag (42) korrespondiert, und wobei die Anschlageinrichtung (60) eingerichtet ist, mittels des mindestens einen Gegenanschlags (62) die unterschiedlichen Drehwinkel oder Drehbereiche festzulegen.

2. Drehbare Verbindung (1; 1a) nach Anspruch 1, **dadurch gekennzeichnet, dass** die Anschlageinrichtung (60) verdrehsicher zu einem der beiden Teile (40, 50), insbesondere zum zweiten Teil (50), an einem der beiden Teile in mindestens zwei unterschiedlichen Drehwinkelpositionen positionierbar ist.

3. Drehbare Verbindung (1; 1 a) nach Anspruch 1 oder 2, **dadurch gekennzeichnet, dass** der erste Teil (40) in axialer Richtung entlang der Drehachse (R) verlagerbar angeordnet ist, und/oder dass die Anschlageinrichtung (60) in axialer Richtung entlang der Drehachse (R) verlagerbar angeordnet ist, insbesondere zusammen mit dem ersten Teil.

4. Drehbare Verbindung (1; 1a) nach einem der vorhergehenden Ansprüche, **dadurch gekennzeichnet, dass** der erste Teil (40) und die Anschlageinrichtung (60) und wahlweise auch der zweite Teil (50) in axialer Richtung am zweiten Verbindungsbauteil (20) axial positionierbar sind.

5. Drehbare Verbindung (1; 1 a) nach einem der vorhergehenden Ansprüche, **dadurch gekennzeichnet, dass** die Anschlageinrichtung (60) derart angeordnet ist, dass eine verdrehsichere Anordnung der Anschlageinrichtung (60) am zweiten Teil durch eine auf die Anschlageinrichtung (60) wirkende Gewichtskraft sichergestellt ist.

6. Drehbare Verbindung (1; 1a) nach einem der vorhergehenden Ansprüche, **dadurch gekennzeichnet, dass** der zweite Teil eine formschlüssige Kontur (54) zum Festlegen einzelner Drehwinkelpositionen aufweist, insbesondere an einer nach innen weisenden Mantelfläche und/oder an einer in die axiale Richtung weisenden Stirnseite, und dass die Anschlageinrichtung eine damit korrespondierende formschlüssige Kontur (64) aufweist, insbesondere an einer in axialer Richtung zum zweiten Teil (50) hin weisenden Stirnseite.

7. Drehbare Verbindung (1; 1 a) nach einem der vorhergehenden Ansprüche, **dadurch gekennzeichnet, dass** der erste Teil (40) und die Anschlageinrichtung (60) zusammen ein Lager bildeen, insbesondere ein Gleitlager.

8. Drehbare Verbindung (1; 1 a) nach einem der vorhergehenden Ansprüche, **dadurch gekennzeichnet, dass** der erste Teil eine an einer Stirnseite, insbesondere an einer zur Anschlageinrichtung hin weisenden Stirnseite, angeordnete Gleitfläche (46) aufweist und eingerichtet ist, mit der Gleitfläche auf der Anschlageinrichtung (60) gleitend zu drehen, und/oder dass die Anschlageinrichtung eine an einer Stirnseite, insbesondere an einer zum ersten Teil hin weisenden Stirnseite, angeordnete Gleitfläche (66) aufweist und eingerichtet ist, den ersten Teil mittels der Gleitfläche für eine gleitende Drehbewegung um die Drehachse zu lagern, wobei die Gleitfläche (46) des ersten Teils und/oder die Gleitfläche (66) der Anschlageinrichtung bevorzugt vollständig umlaufend als Kreisringfläche ausgebildet ist.

9. Drehbare Verbindung (1; 1a) nach einem der vorhergehenden Ansprüche, **dadurch gekennzeichnet, dass** der anpassbare Anschlagmechanismus (30) eingerichtet ist, einen Drehbereich mit einem relativen Drehwinkel größer 360° einzustellen, insbesondere im Bereich von 360° bis 420°.

10. Drehbare Verbindung (1; 1a) nach einem der vorhergehenden Ansprüche, **dadurch gekennzeichnet, dass** der anpassbare Anschlagmechanismus (30) ein Dämpfungselement, insbesondere aus Elastomermaterial, aufweist, welches mit dem zweiten Teil und/oder der Anschlageinrichtung, insbesondere einer stirnseitig in axialer Richtung hervorstehenden formschlüssigen Kontur (64) der Anschlageinrichtung, korrespondiert.

11. Drehbare Verbindung (1; 1 a) nach einem der vorhergehenden Ansprüche, **dadurch gekennzeichnet, dass** die drehbare Verbindung ein Zwischenelement (70) aufweist, welches in axialer Richtung gesehen zwischen dem ersten Teil (40), insbesondere zwischen der Anschlageinrichtung (60), und dem zweiten Verbindungsbauteil (20) angeordnet ist und mindestens eine formschlüssige Kontur (74, 75) zur drehfesten Verbindung mit der Anschlageinrichtung (60) oder dem zweiten Verbindungsbauteil (20) aufweist, wobei bevorzugt an zwei gegenüberliegenden Stirnflächen (76, 77) des Zwischenelements jeweils eine formschlüssige Kontur (74, 75) angeordnet ist.

12. Drehbare Verbindung (1; 1a) nach einem der vorhergehenden Ansprüche, **dadurch gekennzeichnet, dass** das Zwischenelement (70) als Scheibe, insbesondere ringförmige Scheibe ausgebildet ist, und/oder dass das Zwischenelement (70) keilförmig mit einer uneinheitlichen axialen Abmessung ausgebildet ist.

13. Tragsystem für eine Stativvorrichtung (100) zur Anordnung in einem Operationssaal und zum Positionieren einer medizintechnischen Einrichtung im Operationssaal, umfassend eine drehbare Verbindung (1; 1 a) nach einem der Ansprüche 1 bis 12 sowie das erste Verbindungsbauteil (10), insbesondere in Form einer Spindel, und das zweite Verbindungsbauteil (20), insbesondere in Form einer Buchse.

14. Tragsystem nach Anspruch 13, **dadurch gekennzeichnet, dass** das zweite Verbindungsbauteil als Buchse, insbesondere gabelförmige Buchse, ausgebildet ist, wobei zumindest die Anschlageinrichtung (60) und der zweite Teil (50) und bevorzugt auch der erste Teil (40) in der Buchse angeordnet sind, insbesondere zwischen zwei Ringabschnitten (22) der gabelförmigen Buchse, bevorzugt in einem der beiden Ringabschnitte, wobei die drehbare Verbindung (1; 1a) bevorzugt ein Zwischenelement (70) aufweist, welches in die Buchse, insbesondere in einen der beiden Ringabschnitte, eingelegt ist.

15. Stativvorrichtung (100) zur Anordnung in einem Operationssaal und zum Positionieren einer medizintechnischen Einrichtung im Operationssaal, umfassend eine drehbare Verbindung (1; 1a) nach einem der Ansprüche 1 bis 12 oder ein Tragsystem nach einem der Ansprüche 13 oder 14.

## Claims

1. Rotatable connection (1; 1 a) for a stand apparatus (100) to be arranged in an operating room and comprising an adaptable stop mechanism (30) which can be arranged between a first connection component (10) and a second connection component (20) mounted rotatably around a rotational axis relative to the first connection component (20) and configured to define at least two different relative rotational angles of the connection components (10, 20) relative to the one another or at least two different rotational ranges, the adaptable stop mechanism (30) comprising:
- a first part (40) which can be mounted rotationally lockable on the first connection component (10) and features at least one stop (42);
- a second part (50), which can be arranged non-rotatably on the second connection component (20);
the first part (40) being rotatably mounted relative to the second part (50);
**characterized in that** the adaptable stop mechanism (30) comprises a stop device (60) having at least one counter-stop (62) arranged axially between the two parts (40, 50), wherein the at least one counter-stop (62) corresponds to the at least one stop (42) and wherein the stop device (60) is configured to define the different relative rotational angles or rotational ranges by means of the at least one counter-stop (62).

2. Rotatable connection (1; 1 a) according to Claim 1, **characterized in that** the stop device (60) can be positioned on one of the two parts (40, 50) in at least two different rotational angle positions such that it is non-rotatable in relation to one of the two parts, in particular to the second-part (50).

3. Rotatable connection (1; 1 a) according to Claim 1 or 2, **characterized in that** the first part (40) is arranged such that it can be displaced along the rotational axis (R) in axial direction and/or the stop device (60) is arranged such that it can be displaced along the rotational axis (R) in axial direction, in particular jointly with the first part.

4. Rotational connection (1; 1a) according to one of the preceding claims, **characterized in that** the first part (40) and the stop device (60) and optionally also the second part (50) can be positioned axially on the second connection component (20) in axial direction.

5. Rotational connection (1; 1a) according to one of the preceding claims, **characterized in that** the stop device (60) is arranged such that a non-rotatable arrangement of the stop device (60) on the second part is ensured by weight acting on the stop device (60).

6. Rotational connection (1; 1a) according to one of the preceding claims, **characterized in that** the second part has a form-fitting contour (54) for defining individual rotational angle positions, particularly on an inward pointing casing surface and/or on an end face pointing in axial direction and that the stop device has a corresponding form-fitting contour (64), in particular on an end face pointing in axial direction to the second part (50).

7. Rotational connection (1; 1a) according to one of the preceding claims, **characterized in that** the first part (40) and the stop device (60) together form a bearing, in particular a slide bearing.

8. Rotational connection (1; 1a) according to one of the preceding claims, **characterized in that** the first part comprises a sliding surface (46) arranged on an end face, particularly on an end face pointing toward the stop device, and is configured to rotate with the sliding surface on the stop device (60) in a sliding manner and/or that the stop device comprises a sliding surface (66) arranged on an end face, in particular on an end face pointing toward the first part and is configured to mount the first part by means of the sliding surface for a sliding rotational movement around the rotational axis, the sliding surface (46) of the first part and/or the sliding surface (66) of the stop device preferably being designed as a fully continuous annular circular ring surface.

9. Rotational connection (1; 1 a) according to one of the preceding claims, **characterized in that** the adaptable stop mechanism (30) is configured to adjust a rotational area with a relative rotational angle greater than 360°, in particular between 360° and 420°.

10. Rotational connection (1; 1 a) according to one of the preceding claims, **characterized in that** the adaptable stop mechanism (30) comprises a damping element, in particular made of elastomer, which corresponds to the second part and/or the stop device, in particular a form-fitting contour (64) of the stop device projecting from an end face in axial direction.

11. Rotational connection (1; 1 a) according to one of the preceding claims, **characterized in that** the rotatable connection comprises an intermediate element (70) which is arranged, when viewed in axial direction, between the first part (40), in particular between the stop device (60), and the second connection component (20) and at least one form-fitting contour (74, 75) for torque-proof connection with the stop device (60) or the second connection component (20), wherein a form-fitting contour (74, 75) is arranged preferably on each of the two opposite lying end faces (76, 77) of the intermediate element.

12. Rotational connection (1; 1a) according to one of the preceding claims, **characterized in that** the intermediate element (70) is designed as a disc, in particular a ring-shaped disc, and/or the intermediate element (70) is designed as wedge-shaped and of non-uniform axial dimension.

13. Carrier system for a stand apparatus (100) to be arranged in an operating room and for positioning a medical technology device in the operating room comprising a rotatable connection (1; 1a) according to one of the claims 1 through 12 as well as the first connection component (10), in particular in the form of a spindle, and the second connection component (20), in particular in the form of a sleeve.

14. Carrier system according to Claim 13, **characterized in that** the second connection component is designed as a sleeve, in particular a forked sleeve, wherein at least the stop device (60) and the second part (50) and preferably also the first part (40) are arranged in the sleeve, in particular between two ring-shaped segments (22) of the forked sleeve, preferably in one of the two ring-shaped segments, wherein the rotatable connection (1; 1a) preferably comprises an intermediate element (70) which is inserted into the sleeve, in particular into one of the two ring-shaped segments.

15. Stand apparatus (100) for arranging in an operating room and positioning a medical technology device in the operating room, comprising a rotatable connection (1; 1 a) according to one of the Claims 1 through 12 or a carrier system according to one of the Claims 13 or 14.

## Revendications

1. Liaison rotative (1 ; 1a) pour un dispositif à trépied (100) pour l'agencement dans une salle d'opération, comprenant un mécanisme de butée adaptable (30) qui peut être agencé entre un premier composant de liaison (10) et un second composant de liaison (20) logé de manière rotative autour d'un axe de rotation (R) par rapport au premier composant de liaison (10) et aménagé afin de fixer au moins deux angles de rotation relatifs différents des composants de liaison (10, 20) l'un par rapport à l'autre ou au moins deux différentes zones de rotation, dans laquelle le mécanisme de butée adaptable (30) présente :
- une première partie (40) qui peut être logée de manière blocable en rotation sur le premier composant de liaison (10) et présente au moins une butée (42) ;
- une seconde partie (50) qui peut être agencée de manière à ne pas pouvoir tourner sur le second composant de liaison (20) ;
dans laquelle la première partie (40) est logée de manière rotative par rapport à la seconde partie (50) ;
**caractérisée en ce que** le mécanisme de butée adaptable (30) présente un dispositif de butée (60) avec au moins une butée antagoniste (62) qui est agencée axialement entre les deux parties (40, 50), dans laquelle l'au moins une butée antagoniste (62) correspond à l'au moins une butée (42) et dans laquelle le dispositif de butée (60) est aménagé afin de fixer à l'aide de l'au moins une butée antagoniste (62) les différents angles de rotation ou zones de rotation.

2. Liaison rotative (1 ; 1a) selon la revendication 1, **caractérisée en ce que** le dispositif de butée (60) peut être positionné de manière à ne pas pouvoir tourner par rapport à l'une des deux parties (40, 50), en particulier la seconde partie (50), sur une des deux parties dans au moins deux différentes positions d'angle de rotation.

3. Liaison rotative (1 ; 1a) selon la revendication 1 ou 2, **caractérisée en ce que** la première partie (40) est agencée de manière déplaçable dans le sens axial le long de l'axe de rotation (R) et/ou **en ce que** le dispositif de butée (60) est agencé de manière déplaçable dans le sens axial le long de l'axe de rotation (R), en particulier conjointement avec la première partie.

4. Liaison rotative (1 ; 1a) selon l'une quelconque des revendications précédentes, **caractérisée en ce que** la première partie (40) et le dispositif de butée (60) et au choix aussi la seconde partie (50) peuvent être positionnés axialement dans le sens axial sur le second composant de liaison (20).

5. Liaison rotative (1 ; 1a) selon l'une quelconque des revendications précédentes, **caractérisée en ce que** le dispositif de butée (60) est agencé de telle manière qu'un agencement sans possibilité de rotation du dispositif de butée (60) soit assuré sur la seconde partie par une force de poids agissant sur le dispositif de butée (60).

6. Liaison rotative (1 ; 1a) selon l'une quelconque des revendications précédentes, **caractérisée en ce que** la seconde partie présente un contour à complémentarité de formes (54) pour la fixation de positions d'angle de rotation individuelles, en particulier sur une surface enveloppe tournée vers l'intérieur et/ou sur un côté avant tourné dans le sens axial et **en ce que** le dispositif de butée présente un contour (64) à complémentarité de formes correspondant à celui-ci, en particulier sur un côté avant tourné dans le sens axial vers la seconde partie (50).

7. Liaison rotative (1 ; 1a) selon l'une quelconque des revendications précédentes, **caractérisée en ce que** la première partie (40) et le dispositif de butée (60) forment ensemble un palier, en particulier un palier glissant.

8. Liaison rotative (1 ; 1a) selon l'une quelconque des revendications précédentes, **caractérisée en ce que** la première partie présente une surface glissante (46) agencée sur un côté avant, en particulier sur un côté avant tourné vers le dispositif de butée et est aménagée afin de tourner par glissement avec la surface glissante sur le dispositif de butée (60) et/ou **en ce que** le dispositif de butée présente une surface glissante (66) agencée sur un côté avant, en particulier sur un côté avant tourné vers la première partie et est aménagé afin de loger la première partie à l'aide de la surface glissante pour un mouvement rotatif glissant autour de l'axe de rotation, dans laquelle la surface glissante (46) de la première partie et/ou la surface glissante (66) du dispositif de butée est réalisée de préférence complètement tournante comme une surface annulaire et circulaire.

9. Liaison rotative (1 ; 1a) selon l'une quelconque des revendications précédentes, **caractérisée en ce que** le mécanisme de butée adaptable (30) est aménagé afin de régler une zone de rotation avec un angle de rotation relatif supérieur à 360° en particulier dans la plage de 360° à 420°.

10. Liaison rotative (1 ; 1a) selon l'une quelconque des revendications précédentes, **caractérisée en ce que** le mécanisme de butée adaptable (30) présente un élément d'amortissement, en particulier en matériau élastomère qui correspond à la seconde partie et/ou au dispositif de butée, en particulier à un contour à complémentarité de formes (64) dépassant côté avant dans le sens axial du dispositif de butée.

11. Liaison rotative (1 ; 1a) selon l'une quelconque des revendications précédentes, **caractérisée en ce que** la liaison rotative présente un élément intermédiaire (70) qui est agencé vu dans le sens axial entre la première partie (40), en particulier entre le dispositif de butée (60) et le second composant de liaison (20) et présente au moins un contour à complémentarité de formes (74, 75) pour la liaison ne pouvant pas tourner avec le dispositif de butée (60) ou le second composant de liaison (20), dans laquelle de préférence sur deux surfaces avant (76, 77) en regard de l'élément intermédiaire, respectivement un contour à complémentarité de formes (74, 75) est agencé.

12. Liaison rotative (1 ; 1a) selon l'une quelconque des revendications précédentes, **caractérisée en ce que** l'élément intermédiaire (70) est réalisé sous forme de disque, en particulier de disque annulaire et/ou **en ce que** l'élément intermédiaire (70) est
réalisé en forme de coin avec une dimension axiale inégale.

13. Système porteur pour un dispositif à trépied (100) pour l'agencement dans une salle d'opération et pour le positionnement d'un dispositif de technique médicale dans la salle d'opération, comprenant une liaison rotative (1 ; 1a) selon l'une quelconque des revendications 1 à 12, ainsi que le premier composant de liaison (10), en particulier sous la forme d'une broche et le second composant de liaison (20), en particulier sous la forme d'une douille.

14. Système porteur selon la revendication 13, **caractérisé en ce que** le second composant de liaison est réalisé comme une douille, en particulier comme une douille en forme de fourche, dans lequel au moins le dispositif de butée (60) et la seconde partie (50) et de préférence aussi la première partie (40) sont agencés dans la douille, en particulier entre deux sections annulaires (22) de la douille en forme de fourche, de préférence dans l'une des deux sections annulaires, dans lequel la liaison rotative (1 ; 1a) présente de préférence un élément intermédiaire (70) qui est inséré dans la douille, en particulier dans l'une des deux sections annulaires.

15. Dispositif à trépied (100) pour l'agencement dans une salle d'opération et pour le positionnement d'un dispositif de technique médicale dans la salle d'opération comprenant une liaison rotative (1 ; 1a) selon l'une quelconque des revendications 1 à 12 ou un système porteur selon l'une quelconque des revendications 13 ou 14.
